(19) Europäisches Patentamt
European Patent Office
Office européen des brevets

(11) EP 3 524 687 A1

(12) EUROPEAN PATENT APPLICATION
published in accordance with Art. 153(4) EPC

(43) Date of publication:
**14.08.2019 Bulletin 2019/33**

(21) Application number: **17858156.7**

(22) Date of filing: **11.09.2017**

(51) Int Cl.:
***C12Q 1/68*** *(2018.01)* ***G06F 19/22*** *(2011.01)*
***C12N 15/09*** *(2006.01)*

(86) International application number:
**PCT/JP2017/032699**

(87) International publication number:
**WO 2018/066317 (12.04.2018 Gazette 2018/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.10.2016 JP 2016197562**

(71) Applicant: **FUJIFILM Corporation**
**Tokyo 106-8620 (JP)**

(72) Inventor: **TSUJIMOTO, Takayuki**
**Ashigara-kami-gun**
**Kanagawa 258-8538 (JP)**

(74) Representative: **Klunker IP Patentanwälte PartG mbB**
**Destouchesstraße 68**
**80796 München (DE)**

# (54) METHOD FOR DETERMINING NUMBER OF LOCI REQUIRED AND METHOD FOR DETERMINING NUMBER OF SNP LOCI REQUIRED

(57) Provided are a method for determining the number of loci required, and a method for determining the number of SNPs loci required.

EP 3 524 687 A1

SEQUENCE LISTING

<110> Fujifilm Corporation

<120> Method for determining required number of loci

<130> W-6215PCT

<150> JP 2016-197562
<151> 2016-10-05

<160> 62

<170> PatentIn version 3.5

<210> 1
<211> 20
<212> DNA
<213> Artificial Sequence

<220>
<223> SEQUENCE-1

<400> 1
tagccggatg tgggagatgg 20

<210> 2
<211> 20
<212> DNA
<213> Artificial Sequence

<220>
<223> SEQUENCE-2

<400> 2
ccagcattgg aaagatctgg 20

<210> 3
<211> 37
<212> DNA
<213> Artificial Sequence

<220>
<223> 1-F

<400> 3
cgctcttccg atctctgctt cgatgcggac cttctgg 37

<210> 4
<211> 37
<212> DNA
<213> Artificial Sequence

<220>
<223> 1-R

<400> 4
cgctcttccg atctgactct cccacatccg gctatgg 37

<210> 5
<211> 37
<212> DNA
<213> Artificial Sequence

<220>
<223> 2-F

<400> 5
cgctcttccg atctctgttt ccccgaccat aagcttg 37

<210> 6
<211> 37
<212> DNA
<213> Artificial Sequence

<220>
<223> 2-R

<400> 6
cgctcttccg atctgacata cagggctgag agattgg 37

<210> 7
<211> 37
<212> DNA
<213> Artificial Sequence

<220>
<223> 3-F

<400> 7
cgctcttccg atctctgtga taaggtccga actttgg 37

<210> 8
<211> 37
<212> DNA
<213> Artificial Sequence

<220>
<223> 3-R

<400> 8
cgctcttccg atctgacgcg actgcaagag attcgtg 37

<210> 9
<211> 37
<212> DNA
<213> Artificial Sequence

<220>
<223> 4-F

<400> 9
cgctcttccg atctctgatt tgctgctgac cagggtg 37

<210> 10
<211> 37
<212> DNA

<213> Artificial Sequence

<220>
<223> 4-R

<400> 10
cgctcttccg atctgacagg tacagcttcc catctgg 37

<210> 11
<211> 37
<212> DNA
<213> Artificial Sequence

<220>
<223> 5-F

<400> 11
cgctcttccg atctctgccg tgtgtgagat tctcgtg 37

<210> 12
<211> 37
<212> DNA
<213> Artificial Sequence

<220>
<223> 5-R

<400> 12
cgctcttccg atctgacact gctcagggtc ctctgtg 37

<210> 13
<211> 37
<212> DNA
<213> Artificial Sequence

<220>
<223> 6-F

<400> 13
cgctcttccg atctctggta aagcctccag gatgttg 37

<210> 14
<211> 37
<212> DNA
<213> Artificial Sequence

<220>
<223> 6-R

<400> 14
cgctcttccg atctgacctg gcacttgtgc tgactgg 37

<210> 15
<211> 37
<212> DNA
<213> Artificial Sequence

<220>

<223> 7-F

<400> 15
cgctcttccg atctctgcca aagcgcactc acctgtg 37

<210> 16
<211> 37
<212> DNA
<213> Artificial Sequence

<220>
<223> 7-R

<400> 16
cgctcttccg atctgactag ccagtgagag cgaagtg 37

<210> 17
<211> 37
<212> DNA
<213> Artificial Sequence

<220>
<223> 8-F

<400> 17
cgctcttccg atctctgggc ctagaggacg atgcttg 37

<210> 18
<211> 37
<212> DNA
<213> Artificial Sequence

<220>
<223> 8-R

<400> 18
cgctcttccg atctgactgt tgataaccat gccggtg 37

<210> 19
<211> 37
<212> DNA
<213> Artificial Sequence

<220>
<223> 9-F

<400> 19
cgctcttccg atctctgtgc tggacagtga ctcatgg 37

<210> 20
<211> 37
<212> DNA
<213> Artificial Sequence

<220>
<223> 9-R

<400> 20

cgctcttccg atctgaccat tttcctgtcc tggcttg 37

<210> 21
<211> 37
<212> DNA
<213> Artificial Sequence

<220>
<223> 10-F

<400> 21
cgctcttccg atctctgatc cagttcatat gccgttg 37

<210> 22
<211> 37
<212> DNA
<213> Artificial Sequence

<220>
<223> 10-R

<400> 22
cgctcttccg atctgacgcg ttgctgtcat tcctttg 37

<210> 23
<211> 37
<212> DNA
<213> Artificial Sequence

<220>
<223> 11-F

<400> 23
cgctcttccg atctctgagg ttctgctcgt tggcttg 37

<210> 24
<211> 37
<212> DNA
<213> Artificial Sequence

<220>
<223> 11-R

<400> 24
cgctcttccg atctgaccca agaaggacac ggattgg 37

<210> 25
<211> 37
<212> DNA
<213> Artificial Sequence

<220>
<223> 12-F

<400> 25
cgctcttccg atctctgagc ttcccgggaa attagtg 37

<210> 26
<211> 37
<212> DNA
<213> Artificial Sequence

<220>
<223> 12-R

<400> 26
cgctcttccg atctgacgaa ttttaatcgc ccctgtg 37

<210> 27
<211> 37
<212> DNA
<213> Artificial Sequence

<220>
<223> 13-F

<400> 27
cgctcttccg atctctggga ctgcttagat gccgtgg 37

<210> 28
<211> 37
<212> DNA
<213> Artificial Sequence

<220>
<223> 13-R

<400> 28
cgctcttccg atctgacagt caaaagcatg tcagtgg 37

<210> 29
<211> 37
<212> DNA
<213> Artificial Sequence

<220>
<223> 14-F

<400> 29
cgctcttccg atctctgctc tgtggagtcc gtgatgg 37

<210> 30
<211> 37
<212> DNA
<213> Artificial Sequence

<220>
<223> 14-R

<400> 30
cgctcttccg atctgacatg cagtcacagt ggtatgg 37

<210> 31
<211> 37
<212> DNA

<213>  Artificial Sequence

<220>
<223>  15-F

<400>  31
cgctcttccg atctctgggt aatgctgcaa gctctgg                                37

<210>  32
<211>  37
<212>  DNA
<213>  Artificial Sequence

<220>
<223>  15-R

<400>  32
cgctcttccg atctgaccct aggggatcaa gatgtgg                                37

<210>  33
<211>  37
<212>  DNA
<213>  Artificial Sequence

<220>
<223>  16-F

<400>  33
cgctcttccg atctctgatt catcccctga cttcttg                                37

<210>  34
<211>  37
<212>  DNA
<213>  Artificial Sequence

<220>
<223>  16-R

<400>  34
cgctcttccg atctgactat ttgcagcaga tcgatgg                                37

<210>  35
<211>  37
<212>  DNA
<213>  Artificial Sequence

<220>
<223>  17-F

<400>  35
cgctcttccg atctctgcac caacataaat gggattg                                37

<210>  36
<211>  37
<212>  DNA
<213>  Artificial Sequence

<220>

<223>  17-R

<400>  36
cgctcttccg atctgacgcc actgtgctct gtgatgg                                37

<210>  37
<211>  37
<212>  DNA
<213>  Artificial Sequence

<220>
<223>  18-F

<400>  37
cgctcttccg atctctgagt cctgtgagca tctctgg                                37

<210>  38
<211>  37
<212>  DNA
<213>  Artificial Sequence

<220>
<223>  18-R

<400>  38
cgctcttccg atctgactga gtgaattggc aagtttg                                37

<210>  39
<211>  37
<212>  DNA
<213>  Artificial Sequence

<220>
<223>  19-F

<400>  39
cgctcttccg atctctggca gtttgcaggg actcttg                                37

<210>  40
<211>  37
<212>  DNA
<213>  Artificial Sequence

<220>
<223>  19-R

<400>  40
cgctcttccg atctgaccac tccaggttcg ctcatgg                                37

<210>  41
<211>  37
<212>  DNA
<213>  Artificial Sequence

<220>
<223>  20-F

<400>  41

cgctcttccg atctctgctc ctgcctgttt cagggtg 37

<210> 42
<211> 37
<212> DNA
<213> Artificial Sequence

<220>
<223> 20-R

<400> 42
cgctcttccg atctgacaat ctgcacccgg gagtgtg 37

<210> 43
<211> 37
<212> DNA
<213> Artificial Sequence

<220>
<223> 21-F

<400> 43
cgctcttccg atctctggca acagtctggc tttttttg 37

<210> 44
<211> 37
<212> DNA
<213> Artificial Sequence

<220>
<223> 21-R

<400> 44
cgctcttccg atctgacggg atcaggtact gccgttg 37

<210> 45
<211> 37
<212> DNA
<213> Artificial Sequence

<220>
<223> 22-F

<400> 45
cgctcttccg atctctgcaa ggtgctacat gtgctgg 37

<210> 46
<211> 37
<212> DNA
<213> Artificial Sequence

<220>
<223> 22-R

<400> 46
cgctcttccg atctgaccct ttgcagggga atgtttg 37

<210> 47
<211> 37
<212> DNA
<213> Artificial Sequence

<220>
<223> 23-F

<400> 47
cgctcttccg atctctggag cagcgtacca ttgggtg 37

<210> 48
<211> 37
<212> DNA
<213> Artificial Sequence

<220>
<223> 23-R

<400> 48
cgctcttccg atctgaccag tgttctcgct catgtgg 37

<210> 49
<211> 37
<212> DNA
<213> Artificial Sequence

<220>
<223> 24-F

<400> 49
cgctcttccg atctctgtgt ctcccccttt ttagttg 37

<210> 50
<211> 37
<212> DNA
<213> Artificial Sequence

<220>
<223> 24-R

<400> 50
cgctcttccg atctgacttt acctggcttt ggagttg 37

<210> 51
<211> 37
<212> DNA
<213> Artificial Sequence

<220>
<223> 25-F

<400> 51
cgctcttccg atctctggtc agcaagttgg ctactgg 37

<210> 52
<211> 37
<212> DNA

<213> Artificial Sequence

<220>
<223> 25-R

<400> 52
cgctcttccg atctgacagc cttaggctcc catggtg 37

<210> 53
<211> 37
<212> DNA
<213> Artificial Sequence

<220>
<223> 26-F

<400> 53
cgctcttccg atctctgtgc tgagtgcttt ctgattg 37

<210> 54
<211> 37
<212> DNA
<213> Artificial Sequence

<220>
<223> 26-R

<400> 54
cgctcttccg atctgactca cagacgatag ctgtgtg 37

<210> 55
<211> 37
<212> DNA
<213> Artificial Sequence

<220>
<223> 27-F

<400> 55
cgctcttccg atctctgctt ctcctcctgc tgttttg 37

<210> 56
<211> 37
<212> DNA
<213> Artificial Sequence

<220>
<223> 27-R

<400> 56
cgctcttccg atctgaccca aagtgcagga tgtctgg 37

<210> 57
<211> 37
<212> DNA
<213> Artificial Sequence

<220>

<223> 28-F

<400> 57
cgctcttccg atctctggag actcctccca ctggttg 37

<210> 58
<211> 37
<212> DNA
<213> Artificial Sequence

<220>
<223> 28-R

<400> 58
cgctcttccg atctgacaac ccttgccagg tgacttg 37

<210> 59
<211> 37
<212> DNA
<213> Artificial Sequence

<220>
<223> 29-F

<400> 59
cgctcttccg atctctgtct tcagcagcag ctggtgg 37

<210> 60
<211> 37
<212> DNA
<213> Artificial Sequence

<220>
<223> 29-R

<400> 60
cgctcttccg atctgaccca attccttggg tgacttg 37

<210> 61
<211> 37
<212> DNA
<213> Artificial Sequence

<220>
<223> 30-F

<400> 61
cgctcttccg atctctggcg gaacccatgt acctgtg 37

<210> 62
<211> 37
<212> DNA
<213> Artificial Sequence

<220>
<223> 30-R

<400> 62

```
cgctcttccg atctgacaaa cagagcagaa gtgggtg                    37
```

**Description**

## BACKGROUND OF THE INVENTION

1. Field of the Invention

The present invention relates to a method for determining the number of loci required, and a method for determining the number of SNPs loci required.

2. Description of the Related Art

Genetic analysis has become easy to perform by a DNA sequencer and the like developed recently. However, the total base length of a genome is generally enormous, while a sequencer has limited read capacity. Accordingly, a PCR method has become widespread as a technique in which only a required particular gene region is amplified to perform reading on this selected base sequence thereof, thereby efficiently and accurately performing a genetic analysis. In particular, a technique in which a plurality types of primers are supplied to one PCR reaction system at the same time, and a plurality of gene regions are selectively amplified is called multiplex PCR.

Fetal nucleated red blood cells circulating in the maternal blood are rare cells that only about one cell is contained per $10^6$ to $10^7$ cells in the maternal blood. In a case where a plurality of nucleated red blood cells suspected to be a fetal nucleated red blood cell is harvested, the probability of all these harvested nucleated red blood cells being a fetal nucleated red blood cell is extremely low.

In the related art, extracting a genomic DNA from a single nucleated red blood cell, amplifying whole genomic regions using whole genome amplification (WGA), and then enriching a locus of interest by multiplex PCR or hybridization were generally performed.

However, WGA has a problem that amplification biases are large, making it difficult to quantitatively determining the number of chromosomes. Therefore, in recent years, a plurality of loci are PCR-amplified by using a genomic DNA extracted from a single nucleated red blood cell as a template DNA of multiplex PCR instead of enriching the genomic DNA, base sequence information is acquired using a next-generation sequencer, and therefore the number of chromosomes is quantitatively determined or genotyping is performed.

In this case, as the number of loci, which are amplification subjects, increases, influence of one locus on overall performance becomes little, thereby making target performance such as accuracy easy to be achieved.

Therefore, the number of loci as amplification subjects is preferably large. On the other hand, as the number of loci as amplification subjects increases, designing a primer for all loci as amplification subjects becomes difficult, and furthermore, a primer-dimer is easily formed, thereby increasing a possibility of failing PCR amplification.

## SUMMARY OF THE INVENTION

For this reason, demands for a method for determining the number of loci required for achieving target performance are growing in a method in which a plurality of loci in a genomic DNA extracted from a single cell are PCR-amplified by multiplex PCR to acquire base sequence information, thereby quantitatively determining the number of chromosomes.

Accordingly, an object of the present invention is to provide a method for determining the number of loci required for quantitatively determining the number of chromosomes from a single cell.

Another object of the present invention is to provide a method for determining the number of SNPs loci required in a case of determining whether a single cell suspected to be derived from a fetus isolated from a pregnant woman is derived from the fetus or a mother who is the pregnant woman by using SNPs genotyping.

As a result of extensive studies in order to achieve the above objects, the inventors of the present invention have found that it is possible to provide a method for determining the number of loci required or a method for determining the number of SNPs loci required by inputting experimental results and target performance, performing arithmetic processing of the same, and determining the number of loci required or the number of SNPs loci required, and therefore have completed the present invention.

In other words, the present invention are [1] to [9] as follows.

[1] A method for determining the number of loci required for quantitatively determining the number of chromosomes from a single cell, the method comprising:

an experimental result input step of inputting, via input means, the number of loci n on a subject chromosome, and a coefficient of variation x of the number of sequence reads of the subject chromosome, which are experimental results obtained by multiple repeated trials, and storing the same in storage means;

a target performance input step of inputting, via the input means, a coefficient of variation y of the number of sequence reads of the subject chromosome, which is target performance, and storing the same in the storage means;

a number-of-loci-required determination step of allowing calculation means to read out the number of loci n, the coefficient of variation x, and the coefficient of variation y from the storage means and to calculate the number of loci N required from the number of loci n and the coefficient of variation x which are the experimental results and the coefficient of variation y which is the target performance such that the coefficient of variation x becomes equal to or less than y, and storing the same in the storage means; and

a result display step of allowing the calculation means to read out the number of loci N required for quantitatively determining the number of chromosomes from the storage means, and displaying the same on display means.

[2] The method according to [1], further comprising:

an experimental result conversion step of allowing the calculation means to read out the number of loci n on the subject chromosome and a coefficient of variation x' of the number of sequence reads per locus on the subject chromosome from the storage means and to calculate the coefficient of variation x of the number of sequence reads on the subject chromosome by the following equation, and storing the same in the storage means, after the experimental result input step and before the number-of-loci-required determination step, in a case where the coefficient of variation x' of the number of sequence reads per locus on the subject chromosome is input and stored in the storage means in place of the coefficient of variation x of the number of sequence reads of the subject chromosome in the experimental result input step,

$$x = x'/SQRT(n)$$

where, SQRT (n) represents a square root of n.

[3] The method according to [1] or [2], further comprising:

a target performance conversion step of allowing the calculation means to read out a sensitivity $Se_T$, a specificity $Sp_T$, a positive predictive value $PPV_T$, or a negative predictive value $NPV_T$ from the storage means and to convert the same into a target coefficient of variation y of the number of sequence reads on the subject chromosome, and storing the same in the storage means, after the target performance input step and before the number-of-loci-required determination step, in a case where the sensitivity $Se_T$, the specificity $Sp_T$, the positive predictive value $PPV_T$, or the negative predictive value $NPV_T$ is input and stored in the storage means in place of the coefficient of variation y in the target performance input step.

[4] The method according to [3],

in which, in the target performance conversion step,

in a case where the sensitivity $Se_T$ is read out, assuming a cell group having aneuploidy and following a normal distribution, because a proportion of cells determined to have aneuploidy in this cell group is a sensitivity, a target sensitivity $Se_T$ is substituted for the sensitivity to set a coefficient of variation in this case as the target coefficient of variation y,

in a case where the specificity $Sp_T$ is read out, assuming a cell group having aneuploidy and following a normal distribution, because a proportion of cells determined not to have aneuploidy in this cell group is a specificity, a target specificity $Sp_T$ is substituted for the specificity to set a coefficient of variation in this case as the target coefficient of variation y,

in a case where a positive predictive value $PPV_T$ is read out, the positive predictive value is converted into the sensitivity and the specificity using a pre-given prevalence rate and Equation 1, and furthermore, the sensitivity and the specificity are converted into the target coefficient of variation y according to a case where the sensitivity $Se_T$ is read out and a case where the specificity $Sp_T$ is read out, and

in a case where a negative predictive value $NPV_T$ is read out, the negative predictive value is converted into the specificity and the sensitivity using a pre-given prevalence rate and Equation 2, and furthermore, the specificity and the sensitivity are converted into the target coefficient of variation y according to a case where the sensitivity $Se_T$ is read out and a case where the specificity $Sp_T$ is read out.

Equation 1: positive predictive value = sensitivity × prevalence rate/(sensitivity × prevalence rate + (1 - prevalence rate)(1 - specificity))

$$\text{Equation 2: negative predictive value} = \text{specificity} \times (1 - \text{prevalence rate})/(\text{specificity} \times (1 - \text{prevalence rate}) + \text{prevalence rate} \times (1 - \text{sensitivity}))$$

[5] The method according to any one of [1] to [4],
in which, in the number-of-loci-required determination step, the calculation means reads out the number of loci n, the coefficient of variation x, and the coefficient of variation y from the storage means to calculate the number of loci N required by the following equation,

$$N = f(n,x,y) = \text{ceiling}\ (z)$$

$$z = k \times n \times (x/y)^2$$

where, ceiling (z) represents a smallest integer that is equal to or greater than a real number z, and k is a predetermined coefficient of 1.0 to 1.5.
[6] The method according to [5], in which $z = n \times (x/y)^2$.
[7] A method for determining the number of SNPs loci required in a case of determining whether a single cell suspected to be derived from a fetus isolated from a pregnant woman is derived from the fetus or a mother who is the pregnant woman by using SNPs genotyping, the method comprising:

an experimental result input step of inputting, via input means, an average mutation frequency $\nu$ and an allelic dropout rate $\Theta$% of m SNPs, which are obtained by a precedent experiment for analyzing m SNPs loci with m being an integer of 2 or more, and storing the same in storage means;
a target performance input step of inputting a target accuracy $\Psi$% via the input means, and storing the same in the storage means;
a number-of-SNPs-loci-required determination step of allowing calculation means to read out the number of SNPs loci m, a mutation frequency $\nu$, the allelic dropout rate $\Theta$%, and the target accuracy $\Psi$% from the storage means, to obtain a probability $\tau$ that the number of SNPs loci, where a true genotype of a mother and a genotype of a fetal cell are mismatched to be homozygous and heterozygous, is 0 out of m in a case of the allelic dropout rate $\Theta$%, to obtain a probability $\upsilon = 1$ that the number of SNPs loci, where a true genotype of a mother and a genotype of a fetal cell are mismatched to be homozygous and heterozygous, is 0 out of m in a case of the allelic dropout rate $\Theta$%, to calculate $\Phi = 100 \times (\upsilon - \tau)$, and to increase m by 1 to calculate a smallest integer m that satisfies $\Phi \geq \Psi$, and storing the same in the storage means as the number of SNPs loci M required; and
a result display step of allowing the calculation means to read out, from the storage means, the number of SNPs loci M required in a case of determining whether a single cell suspected to be derived from a fetus isolated from a pregnant woman is derived from the fetus or a mother who is the pregnant woman by using SNPs genotyping, and displaying the same on display means.

[8] The method according to claim [7],
in which in the number-of-SNPs-loci-required determination step, the calculation means reads outs the mutation frequency $\nu$, the allelic dropout rate $\Theta$%, and the target accuracy $\Psi$% from the storage means to calculate the number of SNPs loci M required by the following equations,

$$M = h(\nu,\theta,\psi) = \text{ceiling}\ (\chi)$$

$$\chi = \kappa\log(1 - \psi)/\log(1 - \xi)$$

$$\xi = \nu(1 - \nu)(1 - \theta)$$

$$\psi = \Psi/100$$

$$\theta = \Theta/100$$

where, ceiling ($\chi$) represents a smallest integer that is equal to or greater than a real number $\chi$, and $\kappa$ is a predetermined coefficient of 1.0 to 1.5.

[9] The method according to [8], in which $\chi = \log(1 - \psi)/\log(1 - \xi)$.

According to the present invention, it is possible to provide a method for determining the number of loci required for quantitatively determining the number of chromosomes from a single cell. More specifically, it is possible to provide a method for determining the number of loci required for achieving target performance, in a method in which a plurality of loci in a genomic DNA extracted from a single cell are PCR-amplified by multiplex PCR to acquire base sequence information, thereby quantitatively determining the number of chromosomes.

In addition, according to the present invention, it is possible to provide a method for determining the number of SNPs loci required in a case of determining whether a single cell suspected to be derived from a fetus isolated from a pregnant woman is derived from the fetus or a mother who is the pregnant woman by using SNPs genotyping.

Furthermore, primers subjected to multiplex PCR are reduced, and therefore it is possible to reduce costs and to easily design a primer.

## BRIEF DESCRIPTION OF THE DRAWINGS

Fig. 1 is a conceptual diagram showing a configuration of an apparatus for determining the number of loci required used in a method for determining the number of loci required for quantitatively determining the number of chromosomes from a single cell in the present invention.

Fig. 2 is a flow diagram explaining a method for determining the number of loci required for quantitatively determining the number of chromosomes from a single cell in the present invention.

Fig. 3 is a diagram explaining a sensitivity, a specificity, a positive predictive value, and a negative predictive value.

Fig. 4 is a flow diagram explaining a method for determining the number of SNPs loci required in a case of determining whether a single cell suspected to be derived from a fetus isolated from a pregnant woman is derived from the fetus or a mother who is the pregnant woman by using SNPs genotyping in the present invention.

Fig. 5 is a diagram explaining that an apparent genotype becomes heterozygous and homozygous by allelic dropout in a case where a true genotype is heterozygous.

Fig. 6 is a diagram for explaining a first embodiment of a method for designing a primer for PCR-amplifying a locus as an amplification subject.

Fig. 7 is a diagram for explaining a second embodiment of a method for designing a primer for PCR-amplifying a locus as an amplification subject.

Fig. 8 is a diagram for explaining a third embodiment of a method for designing a primer for PCR-amplifying a locus region as an amplification subject.

Fig. 9 is a graph showing a probability distribution obtained by comparing a true genotype of a mother and a genotype of a fetal cell in a case where an ADO rate is 0% in Example 2.

Fig. 10 is a graph showing the probability distribution obtained by comparing the true genotype of the mother and the genotype of the maternal cell in a case where the ADO rate is 0% in Example 2.

Fig. 11 is a graph showing the probability distribution obtained by comparing the true genotype of the mother and the genotype of the fetal cell in a case where the ADO rate is 50% in Example 2.

Fig. 12 is a graph showing the probability distribution obtained by comparing the true genotype of the mother and the genotype of the maternal cell in a case where the ADO rate is 50% in Example 2.

Fig. 13 is a graph showing a probability that the true genotype of the mother, and the genotype of the maternal cell or the genotype of a fetal cell are mismatched to be homozygous and heterozygous at X out of 20 SNPs loci in a case where the ADO rate is 50% in Example 2. A probability that 0 out of 20 SNPs loci are mismatched to be homozygous and heterozygous is 1 in a case of comparing the true genotype of the mother with the genotype of the maternal cell, and a probability that 0 out of 20 SNPs loci are mismatched to be homozygous and heterozygous is about 0.07 in a case of comparing the true genotype of the mother with the genotype of the fetal cell. Therefore, accuracy of mother and child discrimination is about 93%.

Fig. 14 is a graph showing a probability that the true genotype of the mother, and the genotype of the maternal cell or the genotype of the fetal cell are mismatched to be homozygous and heterozygous at X out of 35 SNPs loci in a case where the ADO rate is 50% in Example 2. A probability that 0 out of 35 SNPs loci are mismatched to be homozygous and heterozygous is 1 in a case of comparing the true genotype of the mother with the genotype of the maternal cell, and a probability that 0 out of 35 SNPs loci are mismatched to be homozygous and heterozygous is 0.01 or less in a case of comparing the true genotype of the mother with the genotype of the fetal cell. Therefore, accuracy of mother and child discrimination is 99% or more.

## DESCRIPTION OF THE PREFERRED EMBODIMENTS

In the present invention, in a case where a range is expressed using "to" for numerical values or the like capable of specifying the range, the range includes left and right numerical values of "to," or the like. For example, in a case where "a to b" is expressed for the numerical value a and b (where $a \neq b$), the range thereof includes a and b. In addition, for example, in a case where "$a_i$ to $a_j$" (where i and j are integers satisfying $1 \leq i \leq k$ and $1 \leq j \leq k$, and $i \neq j$) is expressed for permutations $a_1, a_2, \cdots, a_k$ consisting of k elements (where k is an integer satisfying $k \geq 3$), the range thereof includes $a_i$ and $a_j$.

In the present invention, each of the following abbreviations represents the following meanings.

PCR: Polymerase Chain Reaction
DNA: Deoxyribonucleic acid
SNPs: Single Nucleotide Polymorphisms
CV: Coefficient of variation

In addition, in the present invention defines 0° = 1.

Furthermore, a sensitivity, a specificity, a positive predictive value, and a negative predictive value have the following meanings.

The sensitivity, which is indicated by a/(a + c) shown in Fig. 3, refers to a proportion of positively tested portions in a case where a disease is present (prevalence).

The specificity, which is indicated by d/(b + d) shown in Fig. 3, refers to a proportion of negatively tested portions in a case where no disease is present.

The positive predictive value, which is indicated by a/(a + b) shown in Fig. 3, refers to a proportion of a case where a disease is present (prevalence) among positively tested portions. In addition, in a case where the sensitivity and the prevalence rate are given, the positive predictive value can also be determined as positive predictive value = sensitivity × prevalence rate/(sensitivity × prevalence rate + (1 - prevalence rate)(1 - specificity)).

The negative predictive value, which is indicated by d/(c + d) shown in Fig. 3, refers to a proportion a case where no disease is present among negatively tested portions. In addition, in a case where the specificity and the prevalence rate are given, the negative predictive value can also be determined as negative predictive value = specificity × (1 - prevalence rate)/(specificity × (1 - prevalence rate) + prevalence rate × (1 - sensitivity)).

[Method for determining number of loci required]

The "method for determining the number of loci required" according to the present invention is a method for determining the number of loci required for quantitatively determining the number of chromosomes from a single cell, the method comprising the following steps. The following will be explained with reference to Fig. 1 and Fig. 2 as appropriate.

<Experimental result input step S11>

An experimental result input step is a step of inputting, via input means (keyboard) 14, the number of loci n on a subject chromosome, and a coefficient of variation x of the number of sequence reads of the subject chromosome, which are experimental results obtained by multiple repeated trials, and storing the same in storage means (memory) 12 (S11 of Fig. 2).

In this step, it is also possible to use auxiliary input means 15 as input means with respect to hardware.

<<Subject chromosome>>

A subject chromosome is a chromosome of which the number of chromosomes is to be quantitatively determined. The subject chromosome may be any one of an autosomal chromosome and sex chromosome. Examples thereof include chromosome 21, chromosome 18, chromosome 13, chromosome X, chromosome Y, and the like.

Quantitative determination of chromosome 21 is carried out to detect a trisomy of chromosome 21, for example. Examples of diseases caused due to a trisomy of chromosome 21 include Down syndrome.

Quantitative determination of chromosome 18 is carried out to detect a trisomy of chromosome 18, for example.

Examples of diseases caused due to a trisomy of chromosome 18 include Edward syndrome.

Quantitative determination of chromosome 13 is carried out to detect a trisomy of chromosome 13, for example. Examples of diseases caused due to a trisomy of chromosome 13 include Patau syndrome.

Quantitative determination of chromosome X is carried out to detect an extra X chromosome, for example. Examples of diseases caused due to a trisomy of an extra X chromosome include Klinefelter syndrome or the like.

Quantitative determination of chromosome Y is carried out to detect an extra Y chromosome, for example. Examples of diseases caused due to a trisomy of an extra Y chromosome include XYY syndrome or the like.

<<Locus>>

A locus is a genetic locus or a locus, which is not limited to a gene region and also includes a base sequence which does not correspond to a gene or a location of a genetic marker.

In addition, the number of loci, that is, the number of loci, is not particularly limited, and is appropriately set in consideration that it is a trial to provide experimental results for determining the number of loci required.

<<Number of sequence reads>>

The number of sequence reads is the number of sequence reads per subject chromosome, which is obtained by amplifying a locus on the subject chromosome by multiplex PCR and sequencing amplification products with a next-generation sequencer. In other words, the number of sequence reads is a total number of sequence reads per locus, which is obtained by sequencing PCR products obtained by amplifying a locus on the subject chromosome by multiplex PCR. As will be described later, instead of the total number of sequence reads per locus, an average number of sequence reads per locus may be used as the number of sequence reads.

<<Repeated trials>>

The number of repeated trials is not particularly limited, but the number thereof is set in consideration that it is performed to obtain experimental results for determining the number of loci required. For example, the number of repetitions is preferably 3 to 100, more preferably 5 to 50, and even more preferably 10 to 30.

In a trial per one trial, for example, peripheral blood of a pregnant woman is collected, nucleated red blood cells are isolated from the collected peripheral blood, genomic DNA is extracted from the isolated nucleated red blood cells, multiplex PCR is performed using the extracted genomic DNA as a template DNA, and therefore the number of sequence reads of the obtained PCR amplification products is obtained using the next-generation sequencer.

It is possible to obtain an average value $\mu$, variance $\sigma^2$, coefficient of variation CV = $\sigma/\mu$, and the like of the number of sequence reads of the subject chromosome from the number of sequence reads of the subject chromosome obtained by multiple trials.

<Experimental result conversion step S13>

The method for determining the number of loci required according to the embodiment of the present invention may further include an experimental result conversion step.

An experimental result conversion step is a step of allowing calculation means to read out the number of loci n on the subject chromosome and a coefficient of variation x' of the number of sequence reads per locus on the subject chromosome from the storage means and to calculate the coefficient of variation x of the number of sequence reads on the subject chromosome by the following equation, and storing the same in the storage means (S13 of Fig. 2).

$$x = x'/\mathrm{SQRT}\ (n)$$

Here, SQRT (n) represents a square root of n.

The experimental result conversion step is preferably performed after the above-described experimental result input step and before the above-described number-of-loci-required determination step, in a case where the coefficient of variation x' of the number of sequence reads per locus on the subject chromosome is input and stored in the storage means (memory) 12 in place of the coefficient of variation x of the number of sequence reads of the subject chromosome in the experimental result input step.

<Target performance input step S 12>

A target performance input step is a step of inputting, via the input means, a coefficient of variation y of the number of sequence reads of the subject chromosome, which is target performance, and storing the same in the storage means (S12 of Fig. 2).

A sensitivity $Se_T$, a specificity $Sp_T$, a positive predictive value $PPV_T$, or a negative predictive value $NPV_T$ may be input in place of the coefficient of variation y. In this case, a target performance conversion step of converting the sensitivity $Se_T$, the specificity $Sp_T$, the positive predictive value $PPV_T$, or the negative predictive value $NPV_T$ into a target coefficient of variation y is carried out after the target performance input step and before the number-of-loci-required determination step.

The auxiliary input means (mouse) 15 can also be used as auxiliary input means.

<Target performance conversion step S14>

The method for determining the number of loci required according to the embodiment of the present invention may further include a target performance conversion step.

A target performance conversion step is a step of allowing the calculation means to read out the sensitivity $Se_T$, the specificity $Sp_T$, the positive predictive value $PPV_T$, or the negative predictive value $NPV_T$ from the storage means and to convert the same into the target coefficient of variation y of the number of sequence reads on the subject chromosome, and storing the same in the storage means (S14 of Fig. 2).

The target performance conversion step is preferably performed after the above-described target performance input step and before the above-described number-of-loci-required determination step, in a case where the sensitivity $Se_T$, the specificity $Sp_T$, the positive predictive value $PPV_T$, or the negative predictive value $NPV_T$ is input and stored in the storage means in place of the coefficient of variation y in the target performance input step.

A specific method of target performance conversion will be described.

(1) In a case where the sensitivity $Se_T$ is read out

In a case of assuming a cell group having aneuploidy and following a normal distribution, because a proportion of cells determined to have aneuploidy in this cell group is a sensitivity, a target sensitivity $Se_T$ is substituted for the sensitivity to set a coefficient of variation in this case as the target coefficient of variation y.

(2) In a case where the specificity $Sp_T$ is read out

In a case of assuming a cell group not having aneuploidy and following a normal distribution, because a proportion of cells determined not to have aneuploidy in this cell group is a specificity, a target specificity $Sp_T$ is substituted for the specificity to set a coefficient of variation in this case as the target coefficient of variation y.

(3) In a case where the positive predictive value $PPV_T$ is read out

The positive predictive value is converted into the sensitivity and the specificity using a pre-given prevalence rate and Equation 1, and furthermore, the sensitivity and the specificity are converted into the target coefficient of variation y according to a case where the sensitivity $Se_T$ is read out and a case where the specificity $Sp_T$ is read out.

$$\text{Equation 1: positive predictive value} = \text{sensitivity} \times \text{prevalence rate}/(\text{sensitivity} \times \text{prevalence rate} + (1 - \text{prevalence rate})(1 - \text{specificity}))$$

(4) In a case where the negative predictive value $NPV_T$ is read out

The negative predictive value is converted into the specificity and the sensitivity using a pre-given prevalence rate and Equation 2, and furthermore, the specificity and the sensitivity are converted into the target coefficient of variation y according to a case where the sensitivity $Se_T$ is read out and a case where the specificity $Sp_T$ is read out.

Equation 2: negative predictive value = specificity × (1 - prevalence rate)/(specificity × (1 - prevalence rate) + prevalence rate × (1 - sensitivity))

More specifically details are as described below.

A cell group D1 not having aneuploidy (hereinafter referred to as "normal cell group D1") and a cell group D2 having aneuploidy (hereinafter referred to as "abnormal cell group D2") are taken into consideration.

A test value X of the normal cell group D1 follows a normal distribution $N(\mu_1,\sigma_1^2)$, and a test value X of the abnormal cell group D2 follows a normal distribution $N(\mu_2,\sigma_2^2)$.

In this case, $N(\mu_1,\sigma_1^2)$ is given as a probability distribution having a probability density function $f_1(x)$ below.

$$f_1(x) = 1/sqrt(2\pi\sigma_1^2)\cdot epx(-(x - \mu_1)^2/2\sigma_1^2)$$

In addition, $N(\mu_2,\sigma_2^2)$ is given as a probability distribution having a probability density function $f_2(x)$ below.

$$f_2(x) = 1/sqrt(2\pi\sigma_2^2)\cdot epx(-(x - \mu_2)^2/2\sigma_2^2)$$

Here, sqrt (z) represents a square root of z, and exp (z) represents $e^z$. Here, e is the base of natural logarithm (Napier's constant).

A coefficient of variation $CV_1$ of the normal cell group D1 and a coefficient of variation $CV_2$ of the abnormal cell group D2 are as below, respectively.

$$CV_1 = \sigma_1/\mu_1$$

$$CV_2 = \sigma_2/\mu_2$$

(In a case where the group is determined to have aneuploidy in a case where the test value X exceeds a threshold value c (in a case of $X > c$), and the group is determined not to have aneuploidy in a case where the test value X is equal to or less than the threshold value c (in a case of $X \leq c$).)

The sensitivity is a proportion of cells whose test value X exceeds the threshold value c in the abnormal cell group D2, and is expressed by the following equation.

$$sens(c) = \int_c^\infty f_2(x)dx$$

Therefore, $\sigma_2/\mu_2$ obtained by putting a target sensitivity $Se_T$ into the equation of sens(c) is a target coefficient of variation y obtained by converting the target specificity $Sp_T$.

In addition, the specificity is a proportion of cells whose test value X is equal to or less than the threshold value c in the normal cell group D1, and is expressed by the following equation.

$$spec(c) = \int_0^c f_1(x)dx$$

Therefore, $\sigma_1/\mu_1$ obtained by putting a target sensitivity $Se_T$ into the equation of spec(c) is a target coefficient of variation y obtained by converting the target sensitivity $Se_T$.

Furthermore, based on the following expression, it is possible to obtain a target coefficient of variation y by converting the positive predictive value into the sensitivity and the negative predictive value into the specificity, respectively, using a pre-given prevalence rate, and by further converting the converted results into the coefficient of variation.

$$\text{Positive predictive value} = \text{sensitivity} \times \text{prevalence rate}/(\text{sensitivity} \times \text{prevalence rate} + (1 - \text{prevalence rate})(1 - \text{specificity}))$$

$$\text{Negative predictive value} = \text{specificity} \times (1 - \text{prevalence rate})/(\text{specificity} \times (1 - \text{prevalence rate}) + \text{prevalence rate} \times (1 - \text{sensitivity}))$$

As the threshold value c, for example, c such that $f_1(c) = f_2(c)$ can be employed, but is not limited thereto. (In a case where the group is determined to have aneuploidy in a case where the test value X is less than the threshold value c (in a case of $X < c$), and the group is determined not to have aneuploidy in a case where the test value X is equal to or more than the threshold value c (in a case of $X \geq c$).)

The sensitivity is a proportion of cells whose test value X is less than the threshold value c in the abnormal cell group D2, and is expressed by the following equation.

$$sens(c) = \int_0^c f_2(x)dx$$

Therefore, $\sigma_2/\mu_2$ obtained by putting a target sensitivity $Se_T$ into the equation of sens(c) is a target coefficient of variation y obtained by converting the target specificity $Sp_T$.

In addition, the specificity is a proportion of cells whose test value X is equal to or less than the threshold value c in the normal cell group D1, and is expressed by the following equation.

$$spec(c) = \int_c^\infty f_1(x)dx$$

Therefore, $\sigma_1/\mu_1$ obtained by putting a target sensitivity $Se_T$ into the equation of spec(c) is a target coefficient of variation y obtained by converting the target sensitivity $Se_T$.

Furthermore, based on the following expression, it is possible to obtain a target coefficient of variation y by converting the positive predictive value into the sensitivity and the negative predictive value into the specificity, respectively, using a pre-given prevalence rate, and by further converting the converted results into the coefficient of variation.

$$\text{Positive predictive value} = \text{sensitivity} \times \text{prevalence rate}/(\text{sensitivity} \times \text{prevalence rate} + (1 - \text{prevalence rate})(1 - \text{specificity}))$$

$$\text{Negative predictive value} = \text{specificity} \times (1 - \text{prevalence rate})/(\text{specificity} \times (1 - \text{prevalence rate}) + \text{prevalence rate} \times (1 - \text{sensitivity}))$$

As the threshold value c, for example, c such that $f_1(c) = f_2(c)$ can be employed, but is not limited thereto.

<Number-of-loci-required determination step S15>

A number-of-loci-required determination step is a step of allowing calculation means (CPU) 11 to read out the number of loci n, the coefficient of variation x, and the coefficient of variation y from the storage means (memory) 12 and to calculate the number of loci N required from the number of loci n and the coefficient of variation y which are the experimental results and the coefficient of variation y which is the target performance such that the coefficient of variation becomes equal to or less than y, and storing the same in the storage means (memory) 12 (S15 of Fig. 2).

It is preferable that, in the number-of-loci-required determination step, the calculation means 11 read out the number of loci n, the coefficient of variation x, and the coefficient of variation y from the storage means 12 to calculate the number of loci N required by the following equation.

$$N = f(n,x,y) = \text{ceiling}\ (z)$$

$$z = k \times n \times (x/y)^2,$$

preferably

$$z = n \times (x/y)^2$$

Here, ceiling (z) represents the smallest integer that is equal to or greater than a real number z, and k is a predetermined coefficient of 1.0 to 1.5.

<Result display step S16>

A result display step is a step of allowing the calculation means (CPU) 11 to read out the number of loci N required for quantitatively determining the number of chromosomes from the storage means (memory) 12, and displaying the same on display means (monitor) 16 (S16 of Fig. 2).

In addition, the number of loci N required may be output from output means (printer) 17 according to a command from the input means (keyboard) 14 or the auxiliary input means (mouse) 15.

[Apparatus for determining number of loci required]

Next, an apparatus for executing the method for determining the number of loci required of the embodiment of the present invention will be described with reference to Fig. 1.

The apparatus for executing the method for determining the number of loci required of the embodiment of the present invention comprises the calculation means (CPU) 11, the storage means (memory) 12, auxiliary storage means (storage) 13, the input means (keyboard) 14, and the display means (monitor) 16. In addition, this apparatus may further comprise the auxiliary input means (mouse) 15, the output means (printer) 17, and the like.

Each means will be explained.

The input means (keyboard) 14 is means for inputting instructions, data, and the like to the apparatus, and is used for inputting experimental results and target performance.

The auxiliary input means (mouse) 15 is used in place of or together with the input means (keyboard) 14.

The calculation means (CPU) 11 is means for performing arithmetic processing, and is not only used for processing such as controlling of each means, and writing and reading of data but also for calculating the number of loci N required.

The storage means (memory) 12 is used for storing results of arithmetic processing by the calculation means (CPU) 11, or for storing inputs from the input means (keyboard) 14.

The auxiliary storage means (storage) 13 is a storage for storing an operating system, a program for determining the number of loci required, and the like. A part thereof can also be used to expand the storage means.

The display means (display) 16 is one of interfaces with the user, and is used for displaying the number of loci required after calculating the same.

The output means (printer) 17 is used for printing information displayed on the display means (display) 16 according to an instruction from the calculation means (CPU) 11.

[Method for determining number of SNPs loci required]

A "method for determining the number of SNPs loci required" of the embodiment of the present invention is a method for determining the number of SNPs loci required in a case of determining whether a single cell suspected to be derived from a fetus isolated from a pregnant woman is derived from the fetus or a mother who is the pregnant woman by using SNPs genotyping, the method comprising the following steps.

<Experimental result input step S21>

An experimental result input step is a step of inputting, via the input means (keyboard) 14, an average mutation frequency $\nu$ and an allelic dropout rate $\Theta$% of m SNPs, which are obtained by a precedent experiment for analyzing m SNPs loci with m being an integer of 2 or more, and storing the same in the storage means (memory) 12 (S21 of Fig. 4).

In this step, it is also possible to use the auxiliary input means (mouse) 15 as input means with respect to hardware.

<<Number of SNPs loci>>

The above-mentioned m is not particularly limited as long as it is an integer of 2 or more, but the number thereof is set in consideration of being performed as a precedent experiment. For example, the number of SNPs loci is preferably 10 or more, more preferably 15 or more, and even more preferably 20 or more.

<<Mutation frequency>>

A mutation frequency $\nu$ is $0 \leq \nu \leq 1$. In the present invention, the mutation frequency $\nu$ is preferably $0.35 < \nu < 0.65$, more preferably $0.40 < \nu < 0.60$, and even more preferably $0.45 < \nu < 0.55$.

<<Allelic dropout rate>>

An allelic dropout rate (hereinafter referred to as "allelic dropout (ADO) rate") $\Theta$% is generally $0\% \leq \Theta\% \leq 100\%$. It is preferable that the ADO rate become low.

<Target performance input step S22>

A target performance input step is a step of inputting a target accuracy $\Psi$% via the input means (keyboard) 14, and storing the same in the storage means (memory) 12 (S22 in Fig. 4).

<<Target accuracy>>

A target accuracy $\Psi$% is not particularly limited, but is preferably 95.0% or more, more preferably 99.0% or more, and even more preferably 99.9% or more.

<Number-of-SNPs-loci-required determination step S25>

A number-of-SNPs-loci-required determination step is a step of allowing the calculation means (CPU) 11 to read out the number of SNPs loci m, the mutation frequency $\nu$, the allelic dropout rate $\Theta$%, and the target accuracy $\Psi$% from the storage means (memory) 12,
to obtain a probability $\tau$ that the number of SNPs loci, where a true genotype of a mother and a genotype of a fetal cell are mismatched to be homozygous and heterozygous, is 0 out of m in a case of the allelic dropout rate $\Theta$%,
to obtain a probability $\upsilon = 1$ that the number of SNPs loci, where a true genotype of a mother and a genotype of a fetal cell are mismatched to be homozygous and heterozygous, is 0 out of m in a case of the allelic dropout rate $\Theta$%,
to calculate $\Phi = 100 \times (\upsilon - \tau)$, and
to increase m by 1 to obtain the smallest m that satisfies $\Phi \geq \Psi$, and storing the same in the storage means (memory) 12 as the number of SNPs loci M required (S25 of Fig. 4).

It is preferable that, in the number-of-SNPs-loci-required determination step, the calculation means reads out the mutation frequency $\nu$, the allelic dropout rate $\Theta$%, and a target accuracy $\Psi$% from the storage means to calculate the number of SNPs loci M required by the following equations.

$$M = h(\nu,\theta,\psi) = \text{ceiling}\ (\chi)$$

$$\chi = \kappa \log(1 - \psi)/\log(1 - \xi)$$

$$\xi = \nu(1 - \nu)(1 - \theta)$$

$$\psi = \Psi/100$$

$$\theta = \Theta/100$$

Here, ceiling ($\chi$) represents the smallest integer that is equal to or greater than a real number $\chi$, and $\kappa$ is a predetermined coefficient of 1.0 to 1.5.

<Result display step S26>

A result display step is a step of allowing the calculation means (CPU) 11 to read out, from the storage means (memory) 12, the number of SNPs loci required in a case of determining whether a single cell suspected to be derived from a fetus isolated from a pregnant woman is derived from the fetus or a mother who is the pregnant woman by using SNPs genotyping, and displaying the same on display means (monitor) (S26 in Fig. 4).

In addition, the number of loci N required may be output from the output means (printer) 17 according to a command from the input means (keyboard) 14 or the auxiliary input means (mouse) 15.

<Detailed description of method for calculating number of SNPs loci required in number-of-SNPs-loci-required determination step>

Hereinafter, the number of SNPs loci analyzed in the precedent experiment will be referred to as m ($m \geq 20$, m is an integer), a mutation frequency of m SNPs will be referred to as $\nu$ ($0 \leq \nu \leq 1$), an allelic dropout rate will be referred to as $\theta$ ($0 \leq \theta \leq 1$), and a target accuracy will be referred to as $\psi$ ($0 < \psi \leq 1$).

<<Comparison between true genotype of mother and genotype of fetal cell>>

(In a case of an allelic dropout rate 0%)

For SNPs loci having a mutation frequency $\nu$, a true genotype of a mother and a genotype of a fetal cell are as follows.

(1) Probability of homozygous-homozygous match: $p_{11} = 1 - 3\nu(1 - \nu)$
(2) Probability of heterozygous-heterozygous match: $p_{12} = \nu(1 - \nu)$
(3) Probability of heterozygous-homozygous mismatch: $p_{13} = \nu(1 - \nu)$
(4) Probability of homozygous-heterozygous mismatch: $p_{14} = \nu(1 - \nu)$
(5) Probability of homozygous-homozygous mismatch: $p_{15} = 0$

Therefore, in a case where the true genotype of the mother and the genotype of the fetal cell are compared, a probability $P_{14}$ that k out of $\chi$ SNPs loci are homozygous-heterozygous mismatch is as below.

$$P_{14} = {}_{\chi}C_{k} \cdot p_{14}{}^{k} \cdot (1 - p_{14})^{\chi - k}$$

In a case where the number of SNPs loci is represented by a random variable X, the random variable X follows a binomial distribution $B(\chi, p_{14})$ of parameters $\chi, p_{14}$.

(In a case of an allelic dropout rate $\Theta$%)

In a case where an allelic dropout rate is $\Theta$% ($\Theta = 100 \times \theta$), as shown in Fig. 5, in a case where the true genotype is heterozygous (●○), as an apparent genotype, $(100 - \Theta)$% is heterozygous(●○), and $\Theta$% is homozygous (●● and ○○). In a case where there is no chromosome-dependent bias in an allelic dropout, both homozygote (●●) and homozygote (○○) are $\Theta/2$%.

Therefore, taking an allelic dropout rate Θ% into consideration, a probability $q_{14}$ that the true genotype of the mother and the genotype of the fetal cell are homozygous-heterozygous mismatch is as below.

$$q_{14} = \nu(1 - \nu)(1 - \theta)$$

Here,

$$\theta = \Theta/100.$$

In a case where the true genotype of the mother and the genotype of the fetal cell are compared, a probability $Q_{14}$ that k out of $\chi$ SNPs loci are homozygous-heterozygous mismatch is as below

$$Q_{14} = {}_{\chi}C_k \cdot q_{14}{}^{k} \cdot (1 - q_{14})^{\chi-k}$$

A probability $Q_{14}|_{k=0}$ that k = 0, that is, 0 out of $\chi$ SNPs loci are homozygous-heterozygous mismatch is as below.

$$Q_{14}|_{k=0} = {}_{\chi}C_0 \cdot q_{14}{}^{0} \cdot (1 - q_{14})^{\chi} = (1 - q_{14})^{\chi} = (1 - \xi)^{\chi} = \tau(\chi)$$

$$q14 = \xi = \nu(1 - \nu)(1 - \theta)$$

<<Comparison between true genotype of mother and genotype of maternal cell>>

(In a case of an allelic dropout rate 0%)

For SNPs loci having a mutation frequency v, a true genotype of a mother and a genotype of a fetal cell are as follows.

(1) Probability of homozygous-homozygous match: $p_{21} = \nu^2 + (1 - \nu)^2$
(2) Probability of heterozygous-heterozygous match: $p_{22} = 2\nu(1 - \nu)$
(3) Probability of heterozygous-homozygous mismatch: $p_{23} = 0$
(4) Probability that the true genotype of the mother and the genotype of the maternal cell be homozygous-heterozygous mismatch: $p_{24} = 0$
(5) Probability that the true genotype of the mother and the genotype of the maternal cell be homozygous-homozygous mismatch: $p_{25} = 0$

Therefore, in a case where the true genotype of the mother and the genotype of the maternal cell are compared, a probability $P_{24}$ that k out of $\chi$ SNPs loci are homozygous-heterozygous match is as below.

$$P_{24} = {}_{\chi}C_k \cdot p_{24}{}^{k} \cdot (1 - p_{24})^{\chi-k}$$

In a case where the number of SNPs loci is represented by a random variable X, the random variable X follows a binomial distribution $B(\chi,p_{24})$ of parameters $\chi,p_{24}$.

Because $p_{24} = 0$, the above-mentioned $P_{24}$ is as below.

$$P_{24} = {}_{\chi}C_k \cdot 0^{k} \cdot 1^{\chi-k} = {}_{\chi}C_k \cdot 0^{k}$$

(In a case of an allelic dropout rate Θ%)

In a case where an allelic dropout rate is Θ% (Θ = 100 × θ), as shown in Fig. 5, in a case where the true genotype is heterozygous (●○), as an apparent genotype, (100 - Θ)% is heterozygous(●○), and Θ% is homozygous (●● and ○○). In a case where there is no chromosome-dependent bias in an allelic dropout, both homozygote (●●) and homozygote (○○) are Θ/2%.

In a case where the true genotype of the mother is homozygous, an apparent genotype of the maternal cell does not become heterozygous due to allelic dropout. Therefore, a probability $q_{24}$ that the true genotype of the mother and the genotype of the maternal cell are homozygous-heterozygous mismatch is as below.

$q_{24} = 0$

In a case where the true genotype of the mother and the genotype of the maternal cell are compared, a probability $Q_{24}$ that k out of $\chi$ SNPs loci are homozygous-heterozygous mismatch is as below.

$$Q_{24} = {}_{\chi}C_k \cdot q_{14}{}^k \cdot (1 - q_{24})^{\chi - k}$$

A probability $Q_{24}|_{k=0}$ that k = 0, that is, 0 out of $\chi$ SNPs loci are homozygous-heterozygous mismatch is as below.

$$Q_{24}|_{k=0} = {}_{\chi}C_0 \cdot q_{24}{}^0 \cdot (1 - q_{24})^{\chi} = q_{24}{}^0 = 0^0 = 1 = \upsilon(\chi)$$

$\because 0^0 = 1$ was defined.

Accordingly, regarding the true genotype of the mother and the genotype of the maternal cell, a probability that 0 out of m SNPs loci is homozygous-heterozygous mismatch is 1.

An accuracy $\Psi(\chi)$ of mother and child discrimination is as below.

$$\Psi(\chi) = 100 \times (\upsilon(\chi) - \tau(\chi)) = 100 \times (1 - \tau(\chi)) = (1 - (1 - \xi)^{\chi})$$

$$\xi = \nu(1 - \nu)(1 - \theta).$$

As a target accuracy $\Psi$% ($\Psi = 100 \times \psi$), the number of SNPs loci $\chi$ given with the target accuracy is $\psi = 1 - (1 - \xi)^{\chi}$ based on the above equation, and therefore, according to $\log(1 - \xi)^{\chi} = \log(1 - \psi)$, $\chi = \log(1 - \psi)/\log(1 - \xi)$.

$\chi$ may be multiplied by a coefficient $\kappa$ ($1.0 \leq \kappa \leq 1.5$).

The number of SNPs loci M required is obtained by M = ceiling ($\chi$).

[Apparatus for determining number of SNPs required]

Next, an apparatus for executing the method for determining the number of SNPs required of the embodiment of the present invention will be described with reference to Fig. 1.

The apparatus for executing the method for determining the number of SNPs required of the embodiment of the present invention comprises the calculation means (CPU) 11, the storage means (memory) 12, auxiliary storage means (storage) 13, the input means (keyboard) 14, and the display means (monitor) 16. In addition, this apparatus may further comprise the auxiliary input means (mouse) 15, the output means (printer) 17, and the like.

Each means will be explained.

The input means (keyboard) 14 is means for inputting instructions, data, and the like to the apparatus, and is used for inputting experimental results and target performance.

The auxiliary input means (mouse) 15 is used in place of or together with the input means (keyboard) 14.

The calculation means (CPU) 11 is means for performing arithmetic processing, and is not only used for processing such as controlling of each means, and writing and reading of data but also for calculating the number of SNPs loci required.

The storage means (memory) 12 is used for storing results of arithmetic processing by the calculation means (CPU) 11, or for storing inputs from the input means (keyboard) 14.

The auxiliary storage means (storage) 13 is a storage for storing an operating system, a program for determining the number of SNPs loci required, and the like. A part thereof can also be used to expand the storage means.

The display means (display) 16 is one of interfaces with the user, and is used for displaying the number of SNPs required after calculating the same.

The output means (printer) 17 is used for printing information displayed on the display means (display) 16 according to an instruction from the calculation means (CPU) 11.

[Designing of primer subjected to multiplex PCR]

After determining the number of loci required or the number of SNPs loci required, a primer to be subjected to multiplex PCR may be designed by the method described below. Hereinafter, a region is read as a locus or SNPs loci.

<First embodiment of method for designing primer for PCR-amplifying target region>

A first embodiment of the present invention, which is a method for designing a primer for PCR-amplifying a target region, includes the following steps. The following description will refer to Fig. 6 as appropriate.

A subject region selection step (a) for selecting a subject region from a target region (S101 of Fig. 6).

A primer candidate base sequence generation step (b) of generation at least one base sequence of a primer candidate for PCR-amplifying the subject region based on each base sequence in each of vicinity regions at both ends of the subject region on a genomic DNA (S102 in Fig. 6).

A local alignment step (c) of obtaining a local alignment score by performing pairwise local alignment on two base sequences included in each of combinations which are obtainable by selecting base sequences of two primer candidates from the base sequences of the primer candidates generated in the primer candidate base sequence generation step, under a condition that partial sequences to be compared have 3' terminals of the two base sequences (S103 of Fig. 6).

A first stage selection step (d) of performing first stage selection of the base sequence of the primer candidate for PCR-amplifying the subject region based on the local alignment score (S104 in Fig. 6).

A global alignment step (e) of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates selected in the first stage selection step (S105 of Fig. 6).

A second stage selection step (f) of performing second stage selection of the base sequence of the primer candidate for PCR-amplifying the subject region based on the global alignment score (S106 of Fig. 6).

A primer employment step (g) of employing the base sequence of the primer candidate which is selected in both of the first stage selection step and the second stage selection step as the base sequence of the primer for PCR-amplifying the subject region (S107 in Fig. 6).

However, among the above steps (a) to (g), both of the above steps (c) and (d) and both of the above steps (e) and (f) are performed in random order or at the same time. That is, the steps (e) and (f) may be performed after the step (c) and the step (d) are performed, or the steps (c) and (d) may be performed after the step (e) and the step (f) are performed, or the steps (c) and (d), and the steps (e) and (f) may be performed in parallel.

In a case where the steps (c) and (d) are carried out after carrying out the steps (e) and (f), the steps (e) and (c) are preferably the following steps (e') and (c'), respectively.

A global alignment step (e') of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates generated in the primer candidate base sequence generation step.

A local alignment step (c') of obtaining a local alignment score by performing pairwise local alignment on two base sequences included in each of combinations which are obtainable by selecting base sequences of two primer candidates from the base sequences of the primer candidates selected in the second stage selection step, under a condition that partial sequences to be compared have 3' terminals of the two base sequences.

In addition, in a case where the step (c) and the step (d) are carried out in parallel with the step (e) and the step (f), the step (e) is preferably the following step (e').

A global alignment step (e') of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates generated in the primer candidate base sequence generation step.

Furthermore, in a case of designing a primer and in a case where a base sequence of a primer candidate has been generated, a primer is designed (S108, S109, and S110 of Fig. 6) from the primer candidate base sequence generation step (b) (S102 of Fig. 6) or the subject region selection (a) (S101 of Fig. 6) in a case where the base sequence of the primer candidate has not been generated from the local alignment step (c) (S103 of Fig. 6).

<Second embodiment of method for designing primer for PCR-amplifying target region>

A second embodiment of the present invention, which is a method for designing a primer for PCR-amplifying a target region, includes the following steps. The following description will refer to Fig. 7 as appropriate.

A first step of subject region selection ($a_1$) of selecting a first subject region from target regions (S201 of Fig. 7).
A first step of primer candidate base sequence generation ($b_1$) of generating at least one base sequence of a primer candidate for PCR-amplifying the first subject region based on each base sequence in each of vicinity regions at both ends of the first subject region on a genomic DNA (S202 in Fig. 7).
A first step of local alignment ($c_1$) of obtaining a local alignment score by performing pairwise local alignment on two base sequences included in each of combinations which are obtainable by selecting base sequences of two primer candidates from the base sequences of the primer candidates generated in the first step of primer candidate base sequence generation, under a condition that partial sequences to be compared have 3' terminals of the two base sequences (S203 in Fig. 7).
A first step of first stage selection ($d_1$) of performing first stage selection of the base sequence of the primer candidate for PCR-amplifying the first subject region based on the local alignment score (S204 in Fig. 7).
A first step of global alignment ($e_1$) of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates selected in the first step of first stage selection (S205 in Fig. 7).
A first step of second stage selection ($f_1$) of performing second stage selection of the base sequence of the primer candidate for PCR-amplifying the first subject region based on the global alignment score (S206 in Fig. 7).
A first step of primer employment ($g_1$) of employing the base sequence of the primer candidate which is selected in both of the first step of first stage selection and the first step of second stage selection as a base sequence of a primer for PCR-amplifying the first subject region (S207 in Fig. 7).
However, among the above steps ($a_1$) to ($g_1$), both of the above steps ($c_1$) and ($d_1$) and both of the above steps ($e_1$) and ($f_1$) are performed in random order or at the same time. That is, the steps ($e_1$) and ($f_1$) may be performed after the step ($c_1$) and the step ($d_1$) are performed, or the steps ($c_1$) and ($d_1$) may be performed after the step ($e_1$) and the step ($f_1$) are performed, or the steps ($c_1$) and ($d_1$), and the steps ($e_1$) and ($f_1$) may be performed in parallel.
In a case where the steps ($c_1$) and ($d_1$) are carried out after carrying out the steps ($e_1$) and ($f_1$), the steps ($e_1$) and ($c_1$) are preferably the following steps ($e_1$') and ($c_1$'), respectively.
A first step of global alignment ($e_1$') of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates generated in the first step of primer candidate base sequence generation.
A first step of local alignment ($c_1$') of obtaining a local alignment score by performing pairwise local alignment on two base sequences included in each of combinations which are obtainable by selecting base sequences of two primer candidates from the base sequences of the primer candidates selected in the first step of second stage selection, under a condition that partial sequences to be compared have 3' terminals of the two base sequences.
In addition, in a case where the step ($c_1$) and the step ($d_1$) are carried out in parallel with the step ($e_1$) and the step ($f_1$), the step ($e_1$) is preferably the following step ($e_1$').
A first step of global alignment ($e_1$') of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates generated in the first step of primer candidate base sequence generation.
A second step of subject region selection ($a_2$) of selecting a second subject region different from the first subject region from target regions (S211 in Fig. 7).
A second step of primer candidate base sequence generation ($b_2$) of generating at least one base sequence of a primer candidate for PCR-amplifying the second subject region based on each base sequence in each of vicinity regions at both ends of the second subject region on a genomic DNA (S212 in Fig. 7).
A second step of local alignment ($c_2$) of obtaining a local alignment score by performing pairwise local alignment on two base sequences included in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed, under a condition that partial sequences to be compared have 3' terminals of the two base sequences (S213 in Fig. 7).
A second step of first stage selection ($d_2$) of performing first stage selection of the base sequence of the primer candidate for PCR-amplifying the second subject region based on the local alignment score (S214 in Fig. 7).
A second step of global alignment ($e_2$) of obtaining a global alignment score by performing pairwise global alignment on a base sequence, which has a predetermined sequence length and has 3' terminals of two base sequences included in combinations, in each of combinations which are combinations obtainable by selecting base sequences of two primer candidates from the base sequences of the primer candidates selected in the second step of first stage selection and base sequences of a primer already employed, and combinations obtainable by selecting a base sequence of one primer candidate and a base sequence of one primer already employed (S215 in Fig. 7).

A second step of second stage selection ($f_2$) of performing second stage selection of the base sequence of the primer candidate for PCR-amplifying the second subject region based on the global alignment score (S216 in Fig. 7).

A second step of primer employment ($g_2$) of employing the base sequence of the primer candidate which is selected in both of the second step of first stage selection and the second step of second stage selection as a base sequence of a primer for PCR-amplifying the second subject region (S217 in Fig. 7).

However, among the above steps ($a_2$) to ($g_2$), both of the above steps ($c_2$) and ($d_2$) and both of the above steps ($e_2$) and ($f_2$) are performed in random order or at the same time. That is, the steps ($e_2$) and ($f_2$) may be performed after the step ($c_2$) and the step ($d_2$) are performed, or the steps ($c_2$) and ($d_2$) may be performed after the step ($e_2$) and the step ($f_2$) are performed, or the steps ($c_2$) and ($d_2$), and the steps ($e_2$) and ($f_2$) may be performed in parallel.

In a case where the steps ($c_2$) and ($d_2$) are carried out after carrying out the steps ($e_2$) and ($f_2$), the steps ($e_2$) and ($c_2$) are preferably the following steps ($e_2$') and ($c_2$'), respectively.

A second step of global alignment ($e_2$') of obtaining a global alignment score by performing pairwise global alignment on a base sequence, which has a predetermined sequence length and has 3' terminals of two base sequences included in combinations, in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed.

A second step of local alignment ($c_2$') of obtaining a local alignment score by performing pairwise local alignment on two base sequences included in each of combinations which are combinations obtainable by selecting base sequences of two primer candidates from the base sequences of the primer candidates selected in the second step of second stage selection and base sequences of a primer already employed, and combinations obtainable by selecting a base sequence of one primer candidate and a base sequence of one primer already employed, under a condition that partial sequences to be compared have 3' terminals of the two base sequences.

In addition, in a case where the step ($c_2$) and the step ($d_2$) are carried out in parallel with the step ($e_2$) and the step ($f_2$), the step ($e_2$) is preferably the following step ($e_2$').

A second step of global alignment ($e_2$') of obtaining a global alignment score by performing pairwise global alignment on a base sequence, which has a predetermined sequence length and has 3' terminals of two base sequences included in combinations, in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed.

In the case of further designing the primer, steps from the second step of subject region selection ($a_2$) (S211 in Fig. 7) to the second step of primer employment ($g_2$) (S217 in Fig. 7) are repeated (S208 in Fig. 7). That is, in a case where three or more target regions are included, and in case of designing a primer for third and subsequent subject regions, which have not yet been selected from the three or more target regions, each step from the step ($a_2$) to the step ($g_2$) is repeated for the third and subsequent subject regions.

<Third embodiment of method for designing primer for PCR-amplifying target region>

A third embodiment, which is a method for designing a primer for PCR-amplifying a target region, includes the following steps. The following description will refer to Fig. 8 as appropriate.

A subject region multiple selection step (a-0) of selecting a plurality of subject regions from target regions (S301 in Fig. 8).

A primer candidate base sequence multiple generation step (b-0) of generating at least one base sequence of a primer candidate for PCR-amplifying the plurality of subject regions based on each base sequence in each of vicinity regions at both ends of the plurality of subject regions on a genomic DNA (S302 in Fig. 8).

A first local alignment step (c-1) of obtaining a local alignment score by performing pairwise local alignment on two base sequences included in each of combinations which are obtainable by selecting base sequences of two primer candidates from the base sequences of the primer candidates for PCR-amplifying the first subject region among the base sequences of the primer candidates generated in the primer candidate base sequence multiple generation step, while using one of a plurality of subject regions selected in the subject region multiple selection step as the first subject region, under a condition that partial sequences to be compared have 3' terminals of the two base sequences (S303 in Fig. 8).

A first first-stage selection step (d-1) of performing first stage selection of the base sequence of the primer candidate for PCR-amplifying the first subject region based on the local alignment score (S304 in Fig. 8).

A first global alignment step (e-1) of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates selected in the first first-stage selection step (S305 in Fig. 8).

A first second-stage selection step (f-1) of performing second stage selection of the base sequence of the primer candidate for PCR-amplifying the first subject region based on the global alignment score (S306 in Fig. 8).

A first primer employment step (g-1) of employing the base sequence of the primer candidate which is selected in both of the first first-stage selection step and the first second-stage selection step as the base sequence of the primer for PCR-amplifying the first subject region (S307 in Fig. 8).

However, among the above steps (c-1) to (g-1), both of the above steps (c-1) and (d-1) and both of the above steps (e-1) and (f-1) are performed in random order or at the same time. That is, the steps (e-1) and (f-1) may be performed after the step (c-1) and the step (d-1) are performed, or the steps (c-1) and (d-1) may be performed after the step (e-1) and the step (f-1) are performed, or the steps (c-1) and (d-1), and the steps (e-1) and (f-1) may be performed in parallel.

In a case where the steps (c-1) and (d-1) are carried out after carrying out the steps (e-1) and (f-1), the steps (e-1) and (c-1) are preferably the following steps (e'-1) and (c'-1), respectively.

A first global alignment step (e'-1) of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates for PCR-amplifying the first subject region among the base sequences of the primer candidates generated in the primer candidate base sequence multiple generation step, while using one of a plurality of subject regions selected in the subject region multiple selection step as the first subject region.

A first local alignment step (c'-1) of obtaining a local alignment score by performing pairwise local alignment on two base sequences included in each of combinations which are obtainable by selecting base sequences of two primer candidates from base sequences of primer candidates selected in the first second-stage selection step, under a condition that partial sequences to be compared have 3' terminals of the two base sequences.

In addition, in a case where the step (c-1) and the step (d-1) are carried out in parallel with the step (e-1) and the step (f-1), the step (e-1) is preferably the following step (e'-1).

A first global alignment step (e'-1) of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates for PCR-amplifying the first subject region among the base sequences of the primer candidates generated in the primer candidate base sequence multiple generation step, while using one of a plurality of subject regions selected in the subject region multiple selection step as the first subject region.

A second local alignment step (c-2) of obtaining a local alignment score by performing pairwise local alignment on two base sequences included in each of combinations which are combinations obtainable by selecting base sequences of two primer candidates from base sequences of primer candidates for PCR-amplifying the second subject region and a base sequence of the primer already employed among the base sequences of the primer candidates generated in the primer candidate base sequence multiple generation step, while using one excluding the first subject region among a plurality of subject regions selected in the subject region multiple selection step as the second subject region, and combinations obtainable by selecting a base sequence of one primer candidate and a base sequence of one primer already employed, under a condition that partial sequences to be compared have 3' terminals of the two base sequences (S313 in Fig. 8).

A second first-stage selection step (d-2) of performing first stage selection of the base sequence of the primer candidate for PCR-amplifying the second subject region based on the local alignment score (S314 in Fig. 8).

A second global alignment step (e-2) of obtaining a global alignment score by performing pairwise global alignment on a base sequence, which has a predetermined sequence length and has 3' terminals of two base sequences included in combinations, in each of combinations which are combinations obtainable by selecting base sequences of two primer candidates from the base sequences of the primer candidates selected in the second first-stage selection step and base sequences of a primer already employed, and combinations obtainable by selecting a base sequence of one primer candidate and a base sequence of one primer already employed (S315 in Fig. 8).

A second second-stage selection step (f-2) of performing second stage selection of the base sequence of the primer candidate for PCR-amplifying the second subject region based on the global alignment score (S316 in Fig. 8).

A second primer employment step (g-2) of employing the base sequence of the primer candidate which is selected in both of the second first-stage selection step and the second second-stage selection step as the base sequence of the primer for PCR-amplifying the second subject region (S317 in Fig. 8).

However, among the above steps (c-2) to (g-2), both of the above steps (c-2) and (d-2) and both of the above steps (e-2) and (f-2) are performed in random order or at the same time. That is, the steps (e-2) and (f-2) may be performed after the step (c-2) and the step (d-2) are performed, or the steps (c-2) and (d-2) may be performed after the step (e-2) and the step (f-2) are performed, or the steps (c-2) and (d-2), and the steps (e-2) and (f-2) may be performed in parallel.

In a case where the steps (c-2) and (d-2) are carried out after carrying out the steps (e-2) and (f-2), the steps (e-2) and (c-2) are preferably the following steps (e'-2) and (c'-2), respectively.

A second global alignment step (e'-2) of obtaining a global alignment score by performing pairwise global alignment on a base sequence, which has a predetermined sequence length and has 3' terminals of two base sequences included in combinations, in each of combinations which are combinations obtainable by selecting base sequences of two primer candidates from base sequences of primer candidates for PCR-amplifying the second subject region among the base sequences of the primer candidates generated in the primer candidate base sequence multiple generation step, while using one excluding the first subject region among a plurality of subject regions selected in the subject region multiple selection step as the second subject region, and base sequences of a primer already employed, and combinations obtainable by selecting a base sequence of one primer candidate and a base sequence of one primer already employed.

A second local alignment step (c'-2) of obtaining a local alignment score by performing pairwise local alignment on two base sequences included in each of combinations which are combinations obtainable by selecting base sequences of two primer candidates from the base sequences of the primer candidates selected in the second second-stage selection step and base sequences of a primer already employed, and combinations obtainable by selecting a base sequence of one primer candidate and a base sequence of one primer already employed, under a condition that partial sequences to be compared have 3' terminals of the two base sequences.

In addition, in a case where the step (c-2) and the step (d-2) are carried out in parallel with the step (e-2) and the step (f-2), the step (e-2) is preferably the following step (e'-2).

A second global alignment step (e'-2) of obtaining a global alignment score by performing pairwise global alignment on a base sequence, which has a predetermined sequence length and has 3' terminals of two base sequences included in combinations, in each of combinations which are combinations obtainable by selecting base sequences of two primer candidates from base sequences of primer candidates for PCR-amplifying the second subject region among the base sequences of the primer candidates generated in the primer candidate base sequence multiple generation step, while using one excluding the first subject region among a plurality of subject regions selected in the subject region multiple selection step as the second subject region, and base sequences of a primer already employed, and combinations obtainable by selecting a base sequence of one primer candidate and a base sequence of one primer already employed.

In addition, each step from the second local alignment step to the second primer employment step is repeated for third and subsequent subject regions in a case where at least the one target region has three or more target regions, three or more subject regions have been selected in the subject region multiple selection step, and a base sequence of a primer candidate for PCR-amplifying each of the three or more subject regions has been generated in the primer candidate base sequence multiple generation step; and in a case where one excluding the first and second subject regions among a plurality of subject regions which have been selected in the subject region multiple selection step is used as a third subject region, and a base sequence of a primer for PCR-amplifying the third and subsequent subject regions is employed.

## <Description of each step>

For each of the first to third embodiments of the method for designing a primer for PCR-amplifying a target region, each step will be described with reference to Fig. 6 to Fig. 8 as appropriate.

### <<Subject region selection step>>

In the present specification, the subject region selection step S101 (Fig. 6), the first step of subject region selection S201 and the second step of subject region selection S211 (Fig. 7), and the subject region multiple selection step S301 (Fig. 8) will be collectively referred to as a "subject region selection step" in some cases.

(First embodiment: subject region selection step S101)

This step is shown as "subject region selection" (S101) in Fig. 6.

In the first embodiment, the subject region selection step (a) is a step of selecting a subject region from target regions. The method of selection is not particularly limited. For example, in a case where a priority order of designing a primer is assigned to the target regions, a subject region for designing a primer is selected from the target regions according to the order indicated by this priority order.

(Second embodiment: first step of subject region selection S201 and second step of subject region selection S211)

These steps are shown as "first step of subject region selection" (S201) and "second step of subject region selection" (S211) in Fig. 7.

In the second embodiment, the first step of subject region selection ($a_1$) is a step of selecting a first subject region from target regions, and the second step of subject region selection ($a_2$) is a step of selecting a second subject region from target regions which have not yet been selected as a subject region. The method of selection is not particularly limited. For example, in a case where a priority order of designing a primer is assigned to the target regions, a subject region for designing a primer is selected from the target regions according to the order indicated by this priority order.

(Third embodiment: subject region multiple selection step S301)

This step is shown as "subject region multiple selection" (S301) in Fig. 8.

In the third embodiment, the subject region multiple selection step (a-0) is a step of selecting a plurality of subject regions from target regions. The method of selection is not particularly limited. For example, in a case where a priority order of designing a primer is assigned to the target regions, a plurality of subject regions for designing a primer are selected from the target regions according to the order indicated by this priority order.

<<Primer candidate base sequence generation step>>

In the present specification, the primer candidate base sequence generation step S102 (Fig. 6), the first step of primer candidate base sequence generation S202 and the second step of primer candidate base sequence generation S212 (Fig. 7), and the primer candidate base sequence multiple generation step S302 (Fig. 8) will be collectively referred to as a "primer candidate base sequence generation step" in some cases.

(First embodiment: primer candidate base sequence generation step S102)

This step is shown as "primer candidate base sequence generation" (S102) in Fig. 6.

In the first embodiment, the primer candidate base sequence generation step (b) is a step of generating at least one base sequence of a primer candidate for PCR-amplifying the subject region based on each base sequence in each of vicinity regions at both ends of the subject region on a genomic DNA.

(Second embodiment: first step of primer candidate base sequence generation S202 and second step of primer candidate base sequence generation S212)

These steps are shown as "first step of primer candidate base sequence generation" (S202) and "second step of primer candidate base sequence generation" (S212) in Fig. 7.

In the second embodiment, the first step of primer candidate base sequence generation ($b_1$) is a step of generating at least one base sequence of a primer candidate for PCR-amplifying the first subject region based on each base sequence in each of vicinity regions at both ends of the first subject region on a genomic DNA, and the second step of primer candidate base sequence generation ($b_2$) is a step of generating at least one base sequence of a primer candidate for PCR-amplifying the second subject region based on each base sequence in each of vicinity regions at both ends of the second subject region on a genomic DNA.

In the second embodiment, with respect to one subject region, generation of a base sequence of a primer candidate, selection of a primer candidate, and employment of a primer are carried out, and the same steps are repeated for the next one subject region.

(Third embodiment: primer candidate base sequence multiple generation step S302)

This step is shown as "primer candidate base sequence multiple generation" (S302) in Fig. 8.

In the third embodiment, the primer candidate base sequence multiple generation step (b-0) is a step of generating at least one base sequence of a primer candidate for PCR-amplifying the plurality of subject regions based on each base sequence in each of vicinity regions at both ends of the plurality of subject regions on a genomic DNA.

In the third embodiment, a base sequence of a primer candidate is generated for all of the plurality of subject regions, and selection and employment are repeated in subsequent steps.

(Vicinity region)

Each vicinity regions of the subject region at both ends is collectively referred to as regions on the outside of the 5' terminal of the subject region and regions on the outside of the 3' terminal of the subject region. The inside of the subject region is not included in the vicinity regions.
A length of the vicinity region is not particularly limited, but is preferably less than or equal to a length that can be expanded through PCR and more preferably less than or equal to an upper limit of a length of DNA fragment for which amplification is desired. A length facilitating application of concentration selection and/or sequence reading is particularly preferable. A length of the vicinity region may be appropriately changed in accordance with the type of enzyme (DNA polymerase) used for PCR, and the like. A specific length of the vicinity region is preferably about 20 to 500 bases, more preferably about 20 to 300 bases, even more preferably about 20 to 200 bases, and particularly preferably about 50 to 200 bases.

(Design parameter of primer)

In addition, in a case of generating a base sequence of a primer candidate, points, such as the length of a primer, the GC content (referring to a total mole percentage of guanine (G) and cytosine (C) in all nucleic acid bases), a melting temperature (which is a temperature at which 50% of double-stranded DNA is dissociated and becomes single-stranded DNA, and in which Tm is derived from a melting temperature and is referred to as a "Tm value" in some cases, and the unit is "°C"), and deviation of a sequence, to be taken into consideration in a general method for designing a primer are the same.

• Length of primer

A length of primer (the number of nucleotides) is not particularly limited, but is preferably 15 mer to 45 mer, more preferably 20 mer to 45 mer, and even more preferably 20 mer to 30 mer. In a case where a length of primer is within this range, it is easy to design a primer excellent in specificity and amplification efficiency.

• GC content of primer

A GC content of primer is not particularly limited, but is preferably 40 mol% to 60 mol% and more preferably 45 mol% to 55 mol%. In a case where a GC content is within this range, a problem such as a decrease in the specificity and the amplification efficiency due to a high-order structure is less likely to occur.

• Tm value of primer

A Tm value of primer is not particularly limited, but is preferably within a range of 50°C to 65°C and more preferably within a range of 55°C to 65°C.
A difference in Tm values of primer is preferably 5°C or less and more preferably 3°C or less, in a primer pair and a primer set.
Tm values can be calculated using software such as OLIGO Primer Analysis Software (manufactured by Molecular Biology Insights) or Primer 3 (http://www-genome.wi.mit.edu/ftp/distribution/software/).
In addition, Tm values can also be obtained through calculation using the following equation from the number of A's, T's, G's, and C's (which are respectively set as nA, nT, nG, and nC) in a base sequence of a primer.

$$\text{Tm value } (^\circ\text{C}) = 2(\text{nA} + \text{nT}) + 4(\text{nC} + \text{nG})$$

A method for calculating a Tm value is not limited thereto, and a Tm value can be calculated through various well-known methods in the related art.

• Deviation of base of primer

The base sequence of a primer candidate is preferably set as a sequence in which there is no deviation of bases as a whole. For example, it is desirable to avoid a partially GC-rich sequence and a partially AT-rich sequence.
In addition, it is also desirable to avoid continuation of T and/or C (polypyrimidine) and continuation of A and/or G (polypurine).

• 3' Terminal of primer

Furthermore, it is preferable that a 3' terminal base sequence avoids a GC-rich sequence or an AT-rich sequence. G or C is preferable for a 3' terminal base, but is not limited thereto.

<<Specificity-checking step>>

A specificity-checking step of evaluating specificity of a base sequence of a primer candidate may be performed (not shown) based on sequence complementarity with respect to chromosomal DNA of a base sequence of each primer candidate which has been generated in the "primer candidate base sequence generation step."

In the specificity check, in a case where local alignment of a base sequence of chromosomal DNA and a base sequence of a primer candidate is performed and a local alignment score is less than a predetermined value, it is possible to evaluate that the complementarity of the base sequence of the primer candidate with respect to a genomic DNA is low, and the specificity of the base sequence of the primer candidate with respect to a genomic DNA is high. It is desirable to perform local alignment also on a complementary chain of chromosomal DNA. This is because chromosomal DNA is double-stranded whereas the primer is single-stranded DNA. In addition, a base sequence complementary to the base sequence of the primer candidate may be used instead of the base sequence of the primer candidate.

In addition, homology search may be performed on genomic DNA base sequence database using the base sequence of the primer candidate as a query sequence. Examples of a homology search tool include Basic Local Alignment Search Tool (BLAST) (Altschul, S. A., et al., "Basic Local Alignment Search Tool," Journal of Molecular Biology, 1990, October, Vol. 215, pp. 403-410), FASTA (Pearson, W. R., et al., "Improved tools for biological sequence comparison," Proceedings of the National Academy of Sciences of the United States of America, National Academy of Sciences, 1988, April, Vol. 85, pp. 2444-2448), and the like. It is possible to obtain local alignment as a result of performing the homology search.

All of the scores and a threshold value of a local alignment score are not particularly limited, and can be appropriately set in accordance with the length of a base sequence of a primer candidate and/or PCR conditions, and the like. In a case of using a homology search tool, a default value of the homology search tool may be used.

For example, as the scores, it is considered that match (complementary base) = + 1, mismatch (non-complementary base) = - 1, and an indel (insertion and/or deletion) = - 3 are employed, and the threshold value is set to be + 15.

In a case where a base sequence of a primer candidate has complementarity to a base sequence at an unexpected position on chromosomal DNA but has low specificity thereto, in some cases, an artifact is amplified instead of a subject region in a case where PCR is performed using a primer of the base sequence of a primer candidate. Therefore, the case where the base sequence of the primer candidate has complementarity to the base sequence at an unexpected position on chromosomal DNA but has low specificity thereto is excluded.

<<Local alignment step>>

In the present specification, the local alignment step S103 (Fig. 6), the first step of local alignment S203 and the second step of local alignment S213 (Fig. 7), and the first local alignment step S303 and the second local alignment step S313 (Fig. 8) will be collectively referred to as a "local alignment step" in some cases.

(First embodiment: local alignment step S103)

This step is shown as "local alignment" (S103) in Fig. 6.

In the first embodiment, the local alignment step (c) is a step of obtaining a local alignment score by performing pairwise local alignment on two base sequences included in each of combinations which are obtainable by selecting base sequences of two primer candidates from the base sequences of the primer candidates generated in the primer candidate base sequence generation step, under a condition that partial sequences to be compared have 3' terminals of the two base sequences.

(Second embodiment: first step of local alignment S203 and second step of local alignment S213)

These steps are shown as "first step of local alignment" (S203) and "second step of local alignment" (S213) in Fig. 7.

In the second embodiment, the first step of local alignment ($c_1$) is a step of obtaining a local alignment score by performing pairwise local alignment on two base sequences included in each of combinations which are obtainable by selecting base sequences of two primer candidates from the base sequences of the primer candidates generated in the first step of primer candidate base sequence generation, under a condition that partial sequences to be compared have 3' terminals of the two base sequences. The second step of local alignment ($c_2$) is a step of obtaining a local alignment score by performing pairwise local alignment on two base sequences included in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed, under a condition that partial sequences to be compared have 3' terminals of the two base sequences.

(Third embodiment: first local alignment step S303 and second local alignment step S313)

These steps are shown as "first local alignment" (S303) and "second local alignment" (S313) in Fig. 8.

In the third embodiment, the first local alignment step (c-1) is a step of obtaining a local alignment score by performing pairwise local alignment on two base sequences included in each of combinations which are obtainable by selecting base sequences of two primer candidates from the base sequences of the primer candidates for PCR-amplifying the first subject region among base sequences of primer candidates generated in the primer candidate base sequence multiple generation step, under a condition that partial sequences to be compared have 3' terminals of the two base sequences. The second local alignment step (c-2) is a step of obtaining a local alignment score by performing pairwise local alignment on two base sequences included in each of combinations which are combinations obtainable by selecting base sequences of two primer candidates from the base sequences of the primer candidates for PCR-amplifying the second subject region selected among the base sequences of the primer candidates generated in the primer candidate base sequence multiple generation step and base sequences of a primer already employed, and combinations obtainable by selecting a base sequence of one primer candidate and a base sequence of one primer already employed, under a condition that partial sequences to be compared have 3' terminals of the two base sequences.

(Local alignment method)

A combination of base sequences to be subjected to local alignment may be a combination selected while allowing overlapping, or may be a combination selected without allowing overlapping. However, in a case where formability of a primer-dimer between primers of an identical base sequence has not yet been evaluated, the combination selected while allowing overlapping is preferable.

A total number of combinations is "${}_pH_2 = {}_{p+1}C_2 = (p + 1)!/2(p - 1)!$" in a case where the selection is performed while allowing overlapping, and is "${}_pC_2 = p(p - 1)/2$" in a case where the selection is performed without allowing overlapping, in which the total number of base sequences for performing local alignment is set to be p.

Local alignment is alignment which is performed on a partial sequence and in which it is possible to locally check a portion with high complementarity.

However, the local alignment in the present invention is different from local alignment usually performed on a base sequence, and is designed such that partial sequences to be subjected to comparison include the 3' terminals of both base sequences by performing local alignment under the condition that the "partial sequences to be subjected to comparison include the 3' terminals of the base sequences."

Furthermore, in the present invention, an aspect is preferable in which partial sequences to be subjected to comparison include the 3' terminals of both base sequences by performing local alignment under the condition that the "partial sequences to be subjected to comparison include the 3' terminals of the base sequences," that is, the condition that "only alignments in which a partial sequence to be subjected to comparison begins at the 3' terminal of one sequence and ends at the 3' terminal of the other sequence."

Local alignment may be performed by inserting a gap. The gap means insertion and/or deletion (indel) of a base.

In addition, in the local alignment, a case where bases are complementary to each other between base sequence pairs is regarded as a match and a case where bases are not complementary to each other therebetween is regarded as a mismatch.

Alignment is performed such that scores for each of the match, the mismatch, and the indel are given and the total score becomes a maximum. The score may be appropriately set. For example, scores may be set as in Table 1. "-" in Table 1 represents a gap (insertion and/or deletion (indel)).

[Table 1]

| | A | T | G | C | - |
|---|---|---|---|---|---|
| A | -1 | +1 | -1 | -1 | -1 |
| T | +1 | -1 | -1 | -1 | -1 |

(continued)

| | A | T | G | C | - |
|---|---|---|---|---|---|
| G | -1 | -1 | -1 | +1 | -1 |
| C | -1 | -1 | +1 | -1 | -1 |
| - | -1 | -1 | -1 | -1 | |

"-":gap(indel)

For example, it is considered that local alignment is performed on base sequences of SEQ ID NO: 1 and SEQ ID NO: 2 shown in Table 2. Here, scores are as shown in Table 1.

[Table 2]

| | Base sequence (5'→3') |
|---|---|
| SEQ ID NO: 1 | TAGCCGGATGTGGGAGATGG |
| SEQ ID NO: 2 | CCAGCATTGGAAAGATCTGG |

Based on the base sequences of SEQ ID NO: 1 and SEQ ID NO: 2, a dot matrix shown in Table 3 is generated. Specifically, the base sequence of SEQ ID NO: 1 is arranged from the left to the right in an orientation of 5' to 3' and the base sequence of SEQ ID NO: 2 is arranged from the bottom to the top in an orientation of 5' to 3'. "·" is filled in a grid of which bases are complementary to each other, and a dot matrix shown in Table 3 is obtained.

[Table 3]

| | | SEQ ID NO: 1→ | | | | | | | | | | | | | | | | | | | |
|---|---|---|---|---|---|---|---|---|---|---|---|---|---|---|---|---|---|---|---|---|---|
| | | T | A | G | C | C | G | G | A | T | G | T | G | G | G | A | G | A | T | G | G |
| SEQ ID NO: 2→ | G | | | | ● | ● | | | | | | | | | | | | | | | |
| | G | | | | ● | ● | | | | | | | | | | | | | | | |
| | T | | ● | | | | | | ● | | | | | | | ● | | ● | | | |
| | C | | | ● | | | ● | ● | | | ● | | ● | ● | ● | | ● | | | ● | ● |
| | T | | ● | | | | | | ● | | | | | | | ● | | ● | | | |
| | A | ● | | | | | | | | ● | | ● | | | | | | | ● | | |
| | G | | | | ● | ● | | | | | | | | | | | | | | | |
| | A | ● | | | | | | | | ● | | ● | | | | | | | ● | | |
| | A | ● | | | | | | | | ● | | ● | | | | | | | ● | | |
| | A | ● | | | | | | | | ● | | ● | | | | | | | ● | | |
| | G | | | | ● | ● | | | | | | | | | | | | | | | |
| | G | | | | ● | ● | | | | | | | | | | | | | | | |
| | T | | ● | | | | | | ● | | | | | | | ● | | ● | | | |
| | T | | ● | | | | | | ● | | | | | | | ● | | ● | | | |
| | A | ● | | | | | | | | ● | | ● | | | | | | | ● | | |
| | C | | | ● | | | ● | ● | | | ● | | ● | ● | ● | | ● | | | ● | ● |
| | G | | | | ● | ● | | | | | | | | | | | | | | | |
| | A | ● | | | | | | | | ● | | ● | | | | | | | ● | | |
| | C | | | ● | | | ● | ● | | | ● | | ● | ● | ● | | ● | | | ● | ● |
| | C | | | ● | | | ● | ● | | | ● | | ● | ● | ● | | ● | | | ● | ● |

Based on the dot matrix shown in Table 3, alignment (pairwise alignment) of partial sequences shown in Table 4 is obtained (refer to a diagonal line portion of Table 3). In Table 4, matches are represented by "1," and mismatches are represented by ":."

[Table 4]

| | |
|---|---|
| Partial sequence from SEQ ID NO: 1 | 5'- C C G G A T G T G G G A G A T G G -3' |
| | | | : | | | : | : : : : : | | | : |
| Partial sequence from SEQ ID NO: 2 | 3'- G G T C T A G A A A G G T T A C G -5' |

In this (pairwise) alignment, there are two matches, and there are four mismatches. There is no indel (gap).
Therefore, a local alignment score based on this (pairwise) alignment is (+1) × 9 + (-1) × 8 + (-1) × 0 = +1.
The alignment (pairwise alignment) can be obtained not only through the dot matrix method exemplified herein, but also through a dynamic programming method, a word method, or various other methods.

<<First stage selection step>>

In the present specification, the first stage selection step S104 (Fig. 6), the first step of first stage selection S204 and the second step of first stage selection S214 (Fig. 7), and the first first-stage selection step S304 and the second first-stage selection step S314 (Fig. 8) will be collectively referred to as "first stage selection step" in some cases.

(First embodiment: first stage selection step S104)

This step is shown as "first stage selection" (S104) in Fig. 6.
In the first embodiment, the first stage selection step (d) is a step of performing first stage selection of the base sequence of the primer candidate for PCR-amplifying the subject region based on the local alignment score.

(Second embodiment: first step of first stage selection S204 and second step of first stage selection S214)

These steps are shown as "first step of first stage selection" (S204) and "second step of first stage selection" (S214) in Fig. 7.
In the second embodiment, the first step of first stage selection ($d_1$) is a step of performing first stage selection of the base sequence of the primer candidate for PCR-amplifying the first subject region based on the local alignment score. The second step of first stage selection ($d_2$) is a step of performing first stage selection of the base sequence of the primer candidate for PCR-amplifying the second subject region based on the local alignment score.

(Third embodiment: first first-stage selection step S304 and second first-stage selection step S314)

These steps are shown as "first first-stage selection" (S304) and "second first-stage selection" (S314) in Fig. 8.
In the third embodiment, the first first-stage selection step (d-1) is a step of performing first stage selection of the base sequence of the primer candidate for PCR-amplifying the first subject region based on the local alignment score. The second first-stage selection step (d-2) is a step of performing first stage selection of the base sequence of the primer candidate for PCR-amplifying the second subject region based on the local alignment score.

(First stage selection method)

A threshold value (also referred to as a "first threshold value") of the local alignment score is predetermined.
In a case where a local alignment score is less than the first threshold value, it is determined that a combination of these two base sequences has low dimer formability, and the following step is performed.
In contrast, in a case where a local alignment score is greater than or equal to the first threshold value, it is determined that a combination of these two base sequences has high primer-dimer formability, and the following step is not performed on this combination.
The first threshold value is not particularly limited and can be appropriately set. For example, the first threshold value may be set according to PCR conditions such as an amount of genomic DNA used as a template for a polymerase chain reaction.
Here, in the example shown in the above-described "local alignment step," a case where the first threshold value is set to "+ 3" is considered.

In the above-described example, the local alignment score is "+1" and is less than "+ 3," which is the first threshold value. Therefore, it is possible to determine that the combination of the base sequences of SEQ ID NO: 1 and SEQ ID NO: 2 has low primer-dimer formability.

The present step is performed on each of combinations which are obtainable with local alignment scores calculated in the local alignment step S103, the first step of local alignment S203, the second step of local alignment S213, the first local alignment step S303, or the second local alignment step S313.

<<Global alignment step>>

In the present specification, the global alignment step S105 (Fig. 6), the first step of global alignment S205 and the second step of global alignment S215 (Fig. 7), and the first global alignment step S305 and the second global alignment step S315 (Fig. 8) will be collectively referred to as a "global alignment step" in some cases.

(First embodiment: global alignment step S105)

This step is shown as "global alignment" (S105) in Fig. 6.

In the first embodiment, the global alignment step (e) is a step of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates selected in the first stage selection step.

(Second embodiment: first step of global alignment S205 and second step of global alignment S215)

These steps are shown as "first step of global alignment" (S205) and "second step of global alignment" (S215) in Fig. 7.

In the second embodiment, the first step of global alignment ($e_1$) is a step of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates selected in the first step of first stage selection. The second step of global alignment ($e_2$) is a step of obtaining a global alignment score by performing pairwise global alignment on a base sequence, which has a predetermined sequence length and has 3' terminals of two base sequences included in combinations, in each of combinations which are combinations obtainable by selecting base sequences of two primer candidates from the base sequences of the primer candidates selected in the second step of first stage selection and base sequences of a primer already employed, and combinations obtainable by selecting a base sequence of one primer candidate and a base sequence of one primer already employed.

(Third embodiment: first global alignment step S305 and second global alignment step S315)

These steps are shown as "first global alignment" (S305) and "second global alignment" (S315) in Fig. 8.

In the third embodiment, the first global alignment step (e-1) is a step of obtaining a global alignment score by performing pairwise global alignment on a base sequence, which has a predetermined sequence length and has 3' terminals of two base sequences included in combinations, in each of combinations which are obtainable by selecting base sequences of two primer candidates from the base sequences of the primer candidates selected in the first first-stage selection step. The second global alignment step (e-2) is a step of obtaining a global alignment score by performing pairwise global alignment on a base sequence, which has a predetermined sequence length and has 3' terminals of two base sequences included in combinations, in each of combinations which are combinations obtainable by selecting base sequences of two primer candidates from the base sequences of the primer candidates selected in the second first-stage selection step and base sequences of a primer already employed, and combinations obtainable by selecting a base sequence of one primer candidate and a base sequence of one primer already employed.

(Global alignment method)

The global alignment score is obtained by selecting two primers from the group consisting of all of the primer candidates generated in the "primer candidate base sequence generation step" (in a case where the "local alignment step" and the "first stage selection step" are performed first, and in a case where there are combinations of primer candidates in which a local alignment score is less than the first threshold value, all of the primer candidates included in the combinations thereof), and all of the primers that have already been employed (limited to cases where primers that have already been employed are present); and performing pairwise global alignment on a base sequence, which has a predetermined sequence length and has the 3' terminal.

A combination of base sequences to be subjected to global alignment may be a combination selected while allowing overlapping, or may be a combination selected without allowing overlapping. However, in a case where formability of a primer-dimer between primers of an identical base sequence has not yet been evaluated, the combination selected while allowing overlapping is preferable.

A total number of combinations is "$_xH_2 = {}_{x+1}C_2 = (x + 1)!/2(x - 1)!$" in a case where the selection is performed while allowing overlapping, and is "$_xC_2 = x(x - 1)/2$" in a case where the selection is performed without allowing overlapping, in which the total number of base sequences for performing global alignment is set to be x.

Global alignment is an alignment which is performed on the "entire sequence" and in which it is possible to check complementarity of the entire sequence.

However, here, the "entire sequence" refers to the entirety of a base sequence which has a predetermined sequence length and includes the 3' terminal of a base sequence of a primer candidate.

Global alignment may be performed by inserting a gap. The gap means insertion and/or deletion (indel) of a base.

In addition, in the global alignment, a case where bases are complementary to each other between base sequence pairs is regarded as a match and a case where bases are not complementary to each other therebetween is regarded as a mismatch.

Alignment is performed such that scores for each of the match, the mismatch, and the indel are given and the total score becomes a maximum. The score may be appropriately set. For example, scores may be set as in Table 1. "-" in Table 1 represents a gap (insertion and/or deletion (indel)).

For example, it is considered that global alignment is performed on three bases (refer to portions with capital letters) at the 3' terminal of each base sequence of SEQ ID NO: 1 and SEQ ID NO: 2 shown in Table 5. Here, scores are as shown in Table 1.

[Table 5]

| | Base sequence (5'→3') |
|---|---|
| SEQ ID NO: 1 | tagccggatgtgggagaTGG |
| SEQ ID NO: 2 | ccagcattggaaagatcTGG |

In a case of performing global alignment on base sequences of the three bases (portion with capital letters) at the 3' terminal of the base sequence of SEQ ID NO: 1 and the three bases (portion with capital letters) at the 3' terminal of SEQ ID NO: 2 such that the score becomes a maximum, it is possible to obtain alignment (pairwise alignment) shown in Table 6. In Table 6, mismatches are represented by ":."

[Table 6]

| | | | | | |
|---|---|---|---|---|---|
| | 5'- | T | G | G | -3' |
| 3 Bases at 3' terminal of SEQ ID NO: 1 | | : | : | : | |
| 3 bases at 3' terminal of SEQ ID NO: 2 | 3'- | G | G | T | -5' |

In this (pairwise) alignment, there are three mismatches, and there is no matches and indels (gap).

Therefore, a global alignment score based on this (pairwise) alignment is $(+1) \times 0 + (-1) \times 3 + (-1) \times 0 = -3$.

The alignment (pairwise alignment) can be obtained through the dot matrix method a dynamic programming method, a word method, or various other methods.

<<Second stage selection step>>

In the present specification, the second stage selection step S106 (Fig. 6), the first step of second stage selection S206 and the second step of second stage selection S216 (Fig. 7), and the first second-stage selection step S306 and the second second-stage selection step S316 (Fig. 8) will be collectively referred to as a "second stage selection step" in some cases.

(First embodiment: second stage selection step S106)

This step is shown as "second stage selection" (S106) in Fig. 6.

In the first embodiment, the second stage selection step (f) is a step of performing second stage selection of the base sequence of the primer candidate for PCR-amplifying the subject region based on the global alignment score.

(Second embodiment: first step of second stage selection S206 and second step of second stage selection S216)

These steps are shown as "first step of second stage selection" (S206) and "second step of second stage selection" (S216) in Fig. 7.

In the second embodiment, the first step of second stage selection ($f_1$) is a step of performing second stage selection of the base sequence of the primer candidate for PCR-amplifying the first subject region based on the global alignment score. The second step of second stage selection ($f_2$) is a step of performing second stage selection of the base sequence of the primer candidate for PCR-amplifying the second subject region based on the global alignment score.

(Third embodiment: first second-stage selection step S306 and second second-stage selection step S3136)

These steps are shown as "first second-stage selection" (S306) and "second second-stage selection" (S316) in Fig. 8.

In the third embodiment, the first second-stage selection step (f-1) is a step of performing second stage selection of the base sequence of the primer candidate for PCR-amplifying the first subject region based on the global alignment score. The second second-stage selection step (f-2) is a step of performing second stage selection of the base sequence of the primer candidate for PCR-amplifying the second subject region based on the global alignment score.

(Second stage selection method)

A threshold value (also referred to as a "second threshold value") of the global alignment score is predetermined.

In a case where a global alignment score is less than the second threshold value, it is determined that a combination of these two base sequences has low dimer formability, and the following step is performed.

In contrast, in a case where a global alignment score is greater than or equal to the second threshold value, it is determined that a combination of these two base sequences has high dimer formability, and the following step is not performed on this combination.

The second threshold value is not particularly limited and can be appropriately set. For example, the second threshold value may be set according to PCR conditions such as an amount of genomic DNA used as a template for a polymerase chain reaction.

It is possible to set the global alignment score obtained by performing pairwise global alignment on a base sequence which has a predetermined number of bases and includes the 3' terminal of a base sequence of each primer to be less than the second threshold value by setting a base sequence with several bases from the 3' terminal of a primer as an identical base sequence.

Here, in the example shown in the above-described "global alignment step," a case where the second threshold value is set to "+ 3" is considered.

In the above-described example, the global alignment score is "-3" and is less than "+ 3," which is the second threshold value. Therefore, it is possible to determine that the combination of the base sequences of SEQ ID NO: 1 and SEQ ID NO: 2 has low primer-dimer formability.

The present step is performed on each of combinations which are obtainable with global alignment scores calculated in the global alignment step S105, the first step of global alignment S205, the second step of global alignment S215, the first global alignment step S305, or the second global alignment step S315.

In addition, in order to reduce the amount of calculation, it is preferable to first perform both steps of the "global alignment step" and the "second stage selection step" and to perform both steps of the "local alignment step" and the "first stage selection step" on a combination of the base sequences of the primers, which has passed the "second stage selection step." Particularly, as the number of subject regions and the number of base sequences of primer candidates increase, the effect of reducing the amount of calculation is increased, and it is possible to speed up the overall processing.

This is because, since global alignment is performed on a base sequence with a short length called a "predetermined sequence length" in the "global alignment step," the amount of calculation of a global alignment score is smaller than that of a local alignment score which is obtained by searching a partial sequence with high complementarity from the entire base sequence under the condition that the base sequence includes the 3' terminal, and therefore it is possible to speed up the processing. It is known that the global alignment is faster than the local alignment in a case of alignment with respect to a sequence having an identical length in a well-known algorithm.

<<Amplification sequence length-checking step>>

An amplification sequence length-checking step (not shown) of calculating a distance between ends of base sequences of primer candidates on chromosomal DNA, in combinations of the base sequences of the primer candidates for which it has been determined that formability of a primer-dimer is low in the "first stage selection step" and "second stage selection step," and determining whether the distance is within a predetermined range may be performed.
In a case where the distance between the ends of the base sequences is within the predetermined range, it is possible to determine that there is a high possibility that combinations of the base sequences of the primer candidates can appropriately amplify a subject region. The distance between the ends of the base sequences of the primer candidates is not particularly limited, and can be appropriately set in accordance with the PCR condition such as the type of enzyme (DNA polymerase). For example, the distance between the ends of the base sequences of the primer candidates can be set to be within various ranges such as a range of 100 to 200 bases (pair), a range of 120 to 180 bases (pair), a range of 140 to 180 bases (pair), a range of 140 to 160 bases (pair), and a range of 160 to 180 bases (pair).

«Primer employment step»

In the present specification, the primer employment step S107 (Fig. 6), the first step of primer employment S207 and the second step of primer employment S217 (Fig. 7), and the first primer employment step S307 and the second primer employment step S317 (Fig. 8) will be collectively referred to as a "primer employment step" in some cases.

(First embodiment: primer employment step S107)

This step is shown as "primer employment" (S107) in Fig. 6.
In the first embodiment, the primer employment step (g) is a step of employing the base sequence of the primer candidate which is selected in both of the first stage selection step and the second stage selection step as the base sequence of the primer for PCR-amplifying the subject region.

(Second embodiment: first step of primer employment S207 and second step of primer employment S217)

These steps are shown as "first step of primer employment" (S207) and "second step of primer employment" (S217) in Fig. 7.
In the second embodiment, the first step of primer employment ($g_1$) is a step of employing the base sequence of the primer candidate which is selected in both of the first step of first stage selection and the first step of second stage selection as a base sequence of a primer for PCR-amplifying the first subject region. The second step of primer employment ($g_2$) is a step of employing the base sequence of the primer candidate which is selected in both of the second step of first stage selection and the second step of second stage selection as a base sequence of a primer for PCR-amplifying the second subject region.

(Third embodiment: first primer employment step S307 and second primer employment step S317)

These steps are shown as "first primer employment" (S307) and "second primer employment" (S317) in Fig. 8.
In the third embodiment, the first primer employment step (g-1) is a step of employing the base sequence of the primer candidate which is selected in both of the first first-stage selection step and the first second-stage selection step as a base sequence of a primer for PCR-amplifying the first subject region. The second primer employment step (g-2) is a step of employing the base sequence of the primer candidate which is selected in both of the second first-stage selection step and the second second-stage selection step as a base sequence of a primer for PCR-amplifying the second subject region.

(Primer employment method)

In the primer employment step, the base sequence of the primer candidate, in which a local alignment score obtained by performing pairwise local alignment on a base sequence of each primer candidate under a condition that a partial sequence to be subjected to comparison includes the 3' terminal of the base sequence is less than the first threshold value, and a global alignment score obtained by performing pairwise global alignment on a base sequence which has a predetermined number of bases and includes the 3' terminal of the base sequence of each primer candidate is less than the second threshold value, is employed as a base sequence of a primer for amplifying a subject region.
For example, it is considered that base sequences of SEQ ID NO: 1 and SEQ ID NO: 2 shown in Table 7 are employed as base sequences of primers for amplifying a subject region.

[Table 7]

| | Base sequence (5'→3') |
|---|---|
| SEQ ID NO: 1 | TAGCCGGATGTGGGAGATGG |
| SEQ ID NO: 2 | CCAGCATTGGAAAGATCTGG |

As already described, the local alignment score is "+1" and thus is less than "+3," which is the first threshold value, and the global alignment score is "-3" and thus is less than "+3," which is the second threshold value.

Accordingly, it is possible to employ the base sequence of the primer candidate represented by SEQ ID NO: 1 and the base sequence of primer candidate represented by SEQ ID NO: 2 as base sequences of primers for amplifying a subject region.

<<Primer design of other target regions>>

After employing a primer for one target region, primers for other target regions may further be designed.

In the first embodiment, in a case where a base sequence of a primer candidate for other target regions has been already generated in the primer candidate base sequence generation step S102, steps are carried out from the local alignment step S103. In a case where a base sequence of a primer candidate for other target regions has not yet generated, other target region is selected in the subject region selection step S101 because the other target region has not been selected in the subject region selection step S101, and after generating a base sequence of a primer candidate for the other target region in the primer candidate base sequence generation step S102, steps subsequent to the local alignment step S103 are carried out.

In the second embodiment, steps are repeated from when a target region other that the first target region is selected in the second step of subject region selection S211.

In the third embodiment, because the base sequence of the primer candidate for the target region selected in the subject region multiple selection step S301 has already been generated in the primer candidate base sequence multiple generation step S302, steps are repeated from the second local alignment step S313.

<<Characteristic points in designing primer and the like>>

Characteristic points in a method for designing a primer for PCR-amplifying a target region, in the method for designing a primer to be subjected to multiplex PCR of the present invention, are generally as follows: selecting a plurality of specific subject regions, searching vicinity base sequences, checking complementarity with each extracted primer set, selecting base sequences having low complementarity, and thereby obtaining primer groups which include a subject region as an amplification subject and do not become complementary to each other.

Characteristic points in checking the complementarity of a base sequence of a primer are that primer groups are generated such that complementarity of a whole sequence becomes low using local alignment and the complementarity with respect to ends of a base sequence of a primer becomes low using global alignment.

In addition, by using a Tm value range calculated from a target value and an actual value as a Tm value in a case of generating a base sequence of a primer candidate, it is possible to more stably perform PCR amplification of a target region.

Hereinafter, the present invention will be described in more detail using Examples, but is not limited to these Examples.

Examples

[Example 1]

It was considered to examine aneuploidy of chromosome 13, chromosome 18, and chromosome 21 at accuracy of a definitive diagnostic level (sensitivity about 99.9% and specificity about 99.999%) by using a single cell.

As a precedent experiment, when amplification was performed on 30 locus by multiplex PCR and the amplified product was sequenced using a next-generation sequencer to measure the number of sequence reads of a subject chromosome, a total CV of amplification (a coefficient of variation of the number of sequence reads of a subject chromosome) was about 30%.

In order to achieve sensitivity 99.9% and specificity 99.999%, it is necessary to make the total CV of amplification (a coefficient of variation of the number of sequence reads of a subject chromosome) about 6.5%.

The number of loci N required for the above achievement can be obtained by the following equations.

$$N = k \times \text{ceiling}(z)$$

z = the number of loci in precedent experiment × (total CV of amplification in precedent experiment/target CV)$^2$
Here, ceiling (z) represents the smallest integer that is equal to or greater than a real number z, and k represents a predetermined coefficient.

In other words, in this example, conditions are as follows.
The number of loci in precedent experiment = 20
Total CV in precedent experiment = 30%
Target CV = 6.5%
Coefficient k = 1.0 assuming that a deterioration in coefficient of variation of the number of sequence reads per locus due increasing the number of loci does not occur.
Accordingly, $z = 1.0 \times 30 \times (30/6.5)^2 \approx 639.05$, and the number of loci required N = ceiling (z) = 640

When amplification by multiplex PCR was performed on 647 locus exceeding the number of loci required, PCR amplification products are sequenced, and the number of sequence reads of a subject chromosome was measured to calculate a total CV of amplification, the total CV of amplification was 6.5% or less, indicating that a definitive diagnosis is possible at accuracy of a sensitivity of 99.9% and a specificity of 99.999%.

In this example, a coefficient k = 1.0 assuming that a deterioration in coefficient of variation of the number of sequence reads per one locus, which is caused by increasing the number of loci, does not occur, but in a case where a deterioration in coefficient of variation of the number of sequence reads per one locus occurs, the coefficient k is set to, for example, k = 1.1,

$$z = 1.1 \times 30 \times (30/6.5)^2 \approx 702.96$$

The number of loci required N = ceiling(z) = 703 may also be used.

[Table 8]

| Locus | | | | Primer | | |
|---|---|---|---|---|---|---|
| ID | Chromosome No. | Top line: Start coordinate<br>Bottom line: End coordinate | Amplification product size (bp) | Name | Base sequence (5'→3')<br>Top line: Forward/Bottom line: Reverse | SEQ ID NO |
| 1 | 13 | 20763333 | 177 | 1-F | cgctcttccgatctctgCTTCGATGCGGACCTTCTGG | 3 |
| | | 20763509 | | 1-R | cgctcttccgatctgacTCTCCCACATCCGGCTATGG | 4 |
| 2 | 13 | 21205086 | 150 | 2-F | cgctcttccgatctctgTTTCCCCGACCATAAGCTTG | 5 |
| | | 21205235 | | 2-R | cgctcttccgatctgacATACAGGGCTGAGAGATTGG | 6 |
| 3 | 13 | 21619945 | 171 | 3-F | cgctcttccgatctctgTGATAAGGTCCGAACTTTGG | 7 |
| | | 21620115 | | 3-R | cgctcttccgatctgacGCGACTGCAAGAGATTCGTG | 8 |
| 4 | 13 | 23898446 | 180 | 4-F | cgctcttccgatctctgATTTGCTGCTGACCAGGGTG | 9 |
| | | 23898625 | | 4-R | cgctcttccgatctgacAGGTACAGCTTCCCATCTGG | 10 |
| 5 | 13 | 24797765 | 179 | 5-F | cgctcttccgatctctgCCGTGTGTGAGATTCTCGTG | 11 |
| | | 24797943 | | 5-R | cgctcttccgatctgacACTGCTCAGGGTCCTCTGTG | 12 |
| 6 | 13 | 25009017 | 154 | 6-F | cgctcttccgatctctgGTAAAGCCTCCAGGATGTTG | 13 |
| | | 25009170 | | 6-R | cgctcttccgatctgacCTGGCACTTGTGCTGACTGG | 14 |
| 7 | 13 | 25029140 | 162 | 7-F | cgctcttccgatctctgCCAAAGCGCACTCACCTGTG | 15 |
| | | 25029301 | | 7-R | cgctcttccgatctgacTAGCCAGTGAGAGCGAAGTG | 16 |
| 8 | 13 | 25264984 | 163 | 8-F | cgctcttccgatctctgGGCCTAGAGGACGATGCTTG | 17 |
| | | 25265146 | | 8-R | cgctcttccgatctgacTGTTGATAACCATGCCGGTG | 18 |
| 9 | 13 | 25266857 | 164 | 9-F | cgctcttccgatctctgTGCTGGACAGTGACTCATGG | 19 |
| | | 25267020 | | 9-R | cgctcttccgatctgacCATTTTCCTGTCCTGGCTTG | 20 |
| 10 | 13 | 25453371 | 179 | 10-F | cgctcttccgatctctgATCCAGTTCATATGCCGTTG | 21 |
| | | 25453549 | | 10-R | cgctcttccgatctgacGCGTTGCTGTCATTCCTTTG | 22 |
| 11 | 18 | 346690 | 181 | 11-F | cgctcttccgatctctgAGGTTCTGCTCGTTGGCTTG | 23 |
| | | 346870 | | 11-R | cgctcttccgatctgacCCAAGAAGGACACGGATTGG | 24 |
| 12 | 18 | 3075668 | 163 | 12-F | cgctcttccgatctctgAGCTTCCCGGGAAATTAGTG | 25 |
| | | 3075830 | | 12-R | cgctcttccgatctgacGAATTTTAATCGCCCCTGTG | 26 |
| 13 | 18 | 3188929 | 159 | 13-F | cgctcttccgatctctgGGACTGCTTAGATGCCGTGG | 27 |
| | | 3189087 | | 13-R | cgctcttccgatctgacAGTCAAAAGCATGTCAGTGG | 28 |
| 14 | 18 | 3457482 | 179 | 14-F | cgctcttccgatctctgCTCTGTGGAGTCCGTGATGG | 29 |
| | | 3457660 | | 14-R | cgctcttccgatctgacATGCAGTCACAGTGGTATGG | 30 |
| 15 | 18 | 5416011 | 179 | 15-F | cgctcttccgatctctgGGTAATGCTGCAAGCTCTGG | 31 |
| | | 5416189 | | 15-R | cgctcttccgatctgacCCTAGGGGATCAAGATGTGG | 32 |
| 16 | 18 | 5956217 | 179 | 16-F | cgctcttccgatctctgATTCATCCCCTGACTTCTTG | 33 |
| | | 5956395 | | 16-R | cgctcttccgatctgacTATTTGCAGCAGATCGATGG | 34 |
| 17 | 18 | 6943188 | 154 | 17-F | cgctcttccgatctctgCACCAACATAAATGGGATTG | 35 |
| | | 6943341 | | 17-R | cgctcttccgatctgacGCCACTGTGCTCTGTGATGG | 36 |
| 18 | 18 | 6983116 | 180 | 18-F | cgctcttccgatctctgAGTCCTGTGAGCATCTCTGG | 37 |
| | | 6983295 | | 18-R | cgctcttccgatctgacTGAGTGAATTGGCAAGTTTG | 38 |
| 19 | 18 | 8796147 | 177 | 19-F | cgctcttccgatctctgGCAGTTTGCAGGGACTCTTG | 39 |
| | | 8796323 | | 19-R | cgctcttccgatctgacCACTCCAGGTTCGCTCATGG | 40 |
| 20 | 18 | 8798079 | 157 | 20-F | cgctcttccgatctctgCTCCTGCCTGTTTCAGGGTG | 41 |
| | | 8798235 | | 20-R | cgctcttccgatctgacAATCTGCACCCGGGAGTGTG | 42 |
| 21 | 21 | 16340252 | 170 | 21-F | cgctcttccgatctctgGCAACAGTCTGGCTTTTTTG | 43 |
| | | 16340421 | | 21-R | cgctcttccgatctgacGGGATCAGGTACTGCCGTTG | 44 |
| 22 | 21 | 28212733 | 158 | 22-F | cgctcttccgatctctgCAAGGTGCTACATGTGCTGG | 45 |
| | | 28212890 | | 22-R | cgctcttccgatctgacCCTTTGCAGGGGAATGTTTG | 46 |
| 23 | 21 | 28305169 | 153 | 23-F | cgctcttccgatctctgGAGCAGCGTACCATTGGGTG | 47 |
| | | 28305321 | | 23-R | cgctcttccgatctgacCAGTGTTCTCGCTCATGTGG | 48 |
| 24 | 21 | 30408533 | 168 | 24-F | cgctcttccgatctctgTGTCTCCCCCTTTTTAGTTG | 49 |
| | | 30408700 | | 24-R | cgctcttccgatctgacTTTACCTGGCTTTGGAGTTG | 50 |
| 25 | 21 | 30464815 | 165 | 25-F | cgctcttccgatctctgGTCAGCAAGTTGGCTACTGG | 51 |
| | | 30464979 | | 25-R | cgctcttccgatctgacAGCCTTAGGCTCCCATGGTG | 52 |
| 26 | 21 | 31709619 | 160 | 26-F | cgctcttccgatctctgTGCTGAGTGCTTTCTGATTG | 53 |
| | | 31709778 | | 26-R | cgctcttccgatctgacTCACAGACGATAGCTGTGTG | 54 |
| 27 | 21 | 31812672 | 176 | 27-F | cgctcttccgatctctgCTTCTCCTCCTGCTGTTTTG | 55 |
| | | 31812847 | | 27-R | cgctcttccgatctgacCCAAAGTGCAGGATGTCTGG | 56 |
| 28 | 21 | 32253431 | 179 | 28-F | cgctcttccgatctctgGAGACTCCTCCCACTGGTTG | 57 |
| | | 32253609 | | 28-R | cgctcttccgatctgacAACCCTTGCCAGGTGACTTG | 58 |
| 29 | 21 | 32638453 | 151 | 29-F | cgctcttccgatctctgTCTTCAGCAGCAGCTGGTGG | 59 |
| | | 32638603 | | 29-R | cgctcttccgatctgacCCAATTCCTTGGGTGACTTG | 60 |
| 30 | 21 | 33694096 | 155 | 30-F | cgctcttccgatctctgGCGGAACCCATGTACCTGTG | 61 |
| | | 33694250 | | 30-R | cgctcttccgatctgacAAACAGAGCAGAAGTGGGTG | 62 |

[Example 2]

(Preconditions)

It was considered to determine whether cells isolated from peripheral blood of a pregnant woman are derived from a mother or derived from a fetus at an accuracy of 99%.

A SNP type of the mother can be determined without being influenced by noise of single cell specific fusion such as ADO by separately collecting a large amount of cells from the oral cavity.

In addition, it was assumed that the mother and a father are not closely related.

(Precedent experiment)

As a precedent experiment, mother and child discrimination was confirmed by multiplex PCR at SNPs 20 loci.

An average mutation frequency of the above-mentioned SNPs 20 loci was referred to as p. Here, $0 < p < 1$.

At this time, a probability that parent and child have different genotypes is $2pq(p^2 + pq + q^2)$. Here, $q = 1 - p$.

In other words, a probability that SNP types of the mother and child match in all 20 loci is about 0.8%, and therefore mother and child could be distinguished from each other with a probability of 99% or more.

In a case where an allelic dropout rate is 0%, a probability that a true genotype of a mother and a genotype of a fetal cell isolated from peripheral blood are homozygous match, a probability of heterozygous match, a probability of becoming heterozygous-homozygous, a probability of becoming homozygous-heterozygous, and a probability of homozygous mismatch are $1 - 3p(1 - p)$, $p(1 - p)$, $p(1 - p)$, $p(1 - p)$, and 0, respectively, in a case where an average mutation frequency of SNPs is p.

In addition, similarly, in a case where an allelic dropout rate is 0%, a probability that a true genotype of a mother and a genotype of a maternal cell isolated from peripheral blood are homozygous match, a probability of heterozygous match, a probability of becoming heterozygous-homozygous, a probability of becoming homozygous-heterozygous, and a probability of homozygous mismatch are $p^2 + (1 - p)^2$, $2p(1 - p)$, 0, 0, and 0, respectively, in a case where an average mutation frequency of SNPs is p.

Therefore, in a case where an average mutation frequency of SNPs of 20 loci is 0.4596, by binomial distributions, distributions of the number that k out of 20 SNPs of a true genotype of a mother and a genotype of a fetal cell are homozygous match, the number of heterozygous match, the number of heterozygous-homozygous mismatch, the number of homozygous-heterozygous mismatch, and the number of homozygous mismatch, respectively, are as shown in Fig. 9.

Similarly, in a case where an average mutation frequency of SNPs of 20 loci is 0.4596, by binomial distributions, distributions of the number that a true genotype of a mother and a genotype of a maternal cell are homozygous match out of 20, the number of heterozygous match, the number of heterozygous-homozygous mismatch, the number of homozygous-heterozygous mismatch, and the number of homozygous mismatch, respectively, are as shown in Fig. 10.

However, as a result of the precedent experiment, it was found that an ADO rate (allelic dropout rate) was 50%.

Because allelic dropout (ADO) occurs at a probability of 50%, a genotype of a locus where a true genotype is heterozygous is apparently determined to be homozygous at a probability of 50%.

Therefore, in a case where an ADO rate is 50%, distributions of the number that a true genotype of a mother and a genotype of a fetal cell are homozygous match out of 20, the number of heterozygous match, the number of heterozygous-homozygous mismatch, the number of homozygous-heterozygous mismatch, and the number of homozygous mismatch, respectively, are corrected as shown in Fig. 11.

Similarly, in a case where an ADO rate is 50%, distributions of the number that a true genotype of a mother and a genotype of a maternal cell are homozygous match out of 20, the number of heterozygous match, the number of heterozygous-homozygous mismatch, the number of homozygous-heterozygous mismatch, and the number of homozygous mismatch, respectively, are corrected as shown in Fig. 12.

Based on Fig. 11 and Fig. 12, a case where a distribution of the number of becoming homozygous-heterozygous is extracted and superimposed, is as shown in Fig. 13.

Because the maternal cell does not become homozygous-heterozygous, an accuracy of mother and child discrimination in Fig. 13 is equal to an intersection area of the graph from Fig. 11 and the graph from Fig. 12. In this case, mother and child can be distinguished at about 93%.

In a case where the number of SNPs increases, a distribution of the fetal cell moves to the right side of the graph, and therefore an accuracy of mother and child discrimination is improved.

As shown in Fig. 14, in a case where the number of SNPs loci was set to 35, an overlap portion between the graph of the fetal cell and the graph of the maternal cell falls below 0.01, and a discrimination accuracy exceeds 99%.

The above description was applied to the following equations.

$$\Psi(\%) = 100 \times \{1 - (1 - \xi)^{\chi}\} \ (\%)$$

$$\xi = \nu(1 - \nu)(100 - \Theta)/100$$

(here, $\chi$ = the number of SNPs loci, $\nu$ = mutation frequency, and $\Theta$ = ADO rate (%))

An accuracy in a case where a mutation frequency is 0.4596, an ADO rate = 50%, and the number of SNPs loci is 20, becomes about 93% according to the following equations.

$$\xi = 0.4596 \times (1 - 0.4596) \times (100 - 50)/100 = 0.1242$$

$$\Psi(\%) = 100 \times \{1 - (1 - 0.1242)^{20}\} \approx 93\%$$

In addition, an accuracy in a case where a mutation frequency is 0.4596, an ADO rate = 50%, and the number of SNPs loci is 35, becomes about 99% according to the following equations.

$$\Psi(\%) = 100 \times \{1 - (1 - 0.1242)^{35}\} \approx 99\%$$

Explanation of References

11: Calculation means (CPU)
12: Storage means (memory)
13: Auxiliary storage means (storage)
14: Input means (keyboard)
15: Auxiliary input means (mouse)
16: Display means (monitor)
17: Output means (printer)

Sequence Listing

International application No. W-6215 PCT based on International Patent Cooperation Treaty Method for determining the number of loci required JP17032699 20170911-00270209251701919596 Normal 20170911153254201709041454188070_P1AP101_W-_12.app

## Claims

1. A method for determining the number of loci required for quantitatively determining the number of chromosomes from a single cell, the method comprising:

    an experimental result input step of inputting, via input means, the number of loci n on a subject chromosome, and a coefficient of variation x of the number of sequence reads of the subject chromosome, which are experimental results obtained by multiple repeated trials, and storing the same in storage means;
    a target performance input step of inputting, via the input means, a coefficient of variation y of the number of sequence reads of the subject chromosome, which is target performance, and storing the same in the storage means;
    a number-of-loci-required determination step of allowing calculation means to read out the number of loci n, the coefficient of variation x, and the coefficient of variation y from the storage means and to calculate the number of loci N required from the number of loci n and the coefficient of variation x which are the experimental results and the coefficient of variation y which is the target performance such that the coefficient of variation x becomes equal to or less than y, and storing the same in the storage means; and
    a result display step of allowing the calculation means to read out the number of loci N required for quantitatively determining the number of chromosomes from the storage means, and displaying the same on display means.

2. The method according to claim 1, further comprising:
an experimental result conversion step of allowing the calculation means to read out the number of loci n on the subject chromosome and a coefficient of variation x' of the number of sequence reads per locus on the subject chromosome from the storage means and to calculate the coefficient of variation x of the number of sequence reads on the subject chromosome by the following equation, and storing the same in the storage means, after the experimental result input step and before the number-of-loci-required determination step, in a case where the coefficient of variation x' of the number of sequence reads per locus on the subject chromosome is input and stored in the storage means in place of the coefficient of variation x of the number of sequence reads of the subject chromosome in the experimental result input step,

$$x = x'/SQRT\ (n)$$

where, SQRT (n) represents a square root of n.

3. The method according to claim 1 or 2, further comprising:
a target performance conversion step of allowing the calculation means to read out a sensitivity $Se_T$, a specificity $Sp_T$, a positive predictive value $PPV_T$, or a negative predictive value $NPV_T$ from the storage means and to convert the same into a target coefficient of variation y of the number of sequence reads on the subject chromosome, and storing the same in the storage means, after the target performance input step and before the number-of-loci-required determination step, in a case where the sensitivity $Se_T$, the specificity $Sp_T$, the positive predictive value $PPV_T$, or the negative predictive value $NPV_T$ is input and stored in the storage means in place of the coefficient of variation y in the target performance input step.

4. The method according to claim 3,

    wherein, in the target performance conversion step,
    in a case where the sensitivity $Se_T$ is read out, assuming a cell group having aneuploidy and following a normal distribution, because a proportion of cells determined to have aneuploidy in this cell group is a sensitivity, a target sensitivity $Se_T$ is substituted for the sensitivity to set a coefficient of variation in this case as the target coefficient of variation y,
    in a case where the specificity $Sp_T$ is read out, assuming a cell group not having aneuploidy and following a normal distribution, because a proportion of cells determined not to have aneuploidy in this cell group is a specificity, a target specificity $Sp_T$ is substituted for the specificity to set a coefficient of variation in this case as the target coefficient of variation y,
in a case where a positive predictive value $PPV_T$ is read out, the positive predictive value is converted into the sensitivity and the specificity using a pre-given prevalence rate and Equation 1, and furthermore, the sensitivity and the specificity are converted into the target coefficient of variation y according to a case where the sensitivity $Se_T$ is read out and a case where the specificity $Sp_T$ is read out, and
in a case where a negative predictive value $NPV_T$ is read out, the negative predictive value is converted into the specificity and the sensitivity using a pre-given prevalence rate and Equation 2, and furthermore, the specificity and the sensitivity are converted into the target coefficient of variation y according to a case where the sensitivity $Se_T$ is read out and a case where the specificity $Sp_T$ is read out.

Equation 1: positive predictive value = sensitivity × prevalence rate/(sensitivity × prevalence rate + (1 - prevalence rate)(1 - specificity))

Equation 2: negative predictive value = specificity × (1 - prevalence rate)/(specificity × (1 - prevalence rate) + prevalence rate × (1 - sensitivity))

**5.** The method according to any one of claims 1 to 4,
wherein, in the number-of-loci-required determination step, the calculation means reads out the number of loci n, the coefficient of variation x, and the coefficient of variation y from the storage means to calculate the number of loci N required by the following equation,

$$N = f(n,x,y) = \text{ceiling}\ (z)$$

$$z = k \times n \times (x/y)^2$$

where, ceiling (z) represents a smallest integer that is equal to or greater than a real number z, and k is a predetermined coefficient of 1.0 to 1.5.

**6.** The method according to claim 5,
wherein

$$z = n \times (x/y)^2.$$

**7.** A method for determining the number of SNPs loci required in a case of determining whether a single cell suspected to be derived from a fetus isolated from a pregnant woman is derived from the fetus or a mother who is the pregnant woman by using SNPs genotyping, the method comprising:

an experimental result input step of inputting, via input means, an average mutation frequency $\nu$ and an allelic dropout rate $\Theta$% of m SNPs, which are obtained by a precedent experiment for analyzing m SNPs loci with m being an integer of 2 or more, and storing the same in storage means;
a target performance input step of inputting a target accuracy $\Psi$% via the input means, and storing the same in the storage means;
a number-of-SNPs-loci-required determination step of allowing calculation means to read out the number of SNPs loci m, a mutation frequency $\nu$, the allelic dropout rate $\Theta$%, and the target accuracy $\Psi$% from the storage means, to obtain a probability $\tau$ that the number of SNPs loci, where a true genotype of a mother and a genotype of a fetal cell are mismatched to be homozygous and heterozygous, is 0 out of m in a case of the allelic dropout rate $\Theta$%, to obtain a probability $\upsilon$ = 1 that the number of SNPs loci, where a true genotype of a mother and a genotype of a fetal cell are mismatched to be homozygous and heterozygous, is 0 out of m in a case of the allelic dropout rate $\Theta$%, to calculate $\Phi = 100 \times (\upsilon - \tau)$, and to increase m by 1 to calculate a smallest integer m that satisfies $\Phi \geq \Psi$, and storing the same in the storage means as the number of SNPs loci M required; and

a result display step of allowing the calculation means to read out, from the storage means, the number of SNPs loci M required in a case of determining whether a single cell suspected to be derived from a fetus isolated from a pregnant woman is derived from the fetus or a mother who is the pregnant woman by using SNPs genotyping, and displaying the same on display means.

**8.** The method according to claim 7,
wherein, in the number-of-SNPs-loci-required determination step, the calculation means reads outs the mutation frequency $\nu$, the allelic dropout rate $\Theta$%, and the target accuracy $\Psi$% from the storage means to calculate the number of SNPs loci M required by the following equations,

$$M = h(\nu,\theta,\psi) = \text{ceiling}\ (\chi)$$

$$\chi = \kappa\log(1 - \psi)/\log(1 - \xi)$$

$$\xi = \nu(1 - \nu)(1 - \theta)$$

$$\psi = \Psi/100$$

$$\theta = \Theta/100$$

where, ceiling ($\chi$) represents a smallest integer that is equal to or greater than a real number $\chi$, and $\kappa$ is a predetermined coefficient of 1.0 to 1.5.

**9.** The method according to claim 8,
wherein

$$\chi = \log(1 - \psi)/\log(1 - \xi).$$

FIG. 1

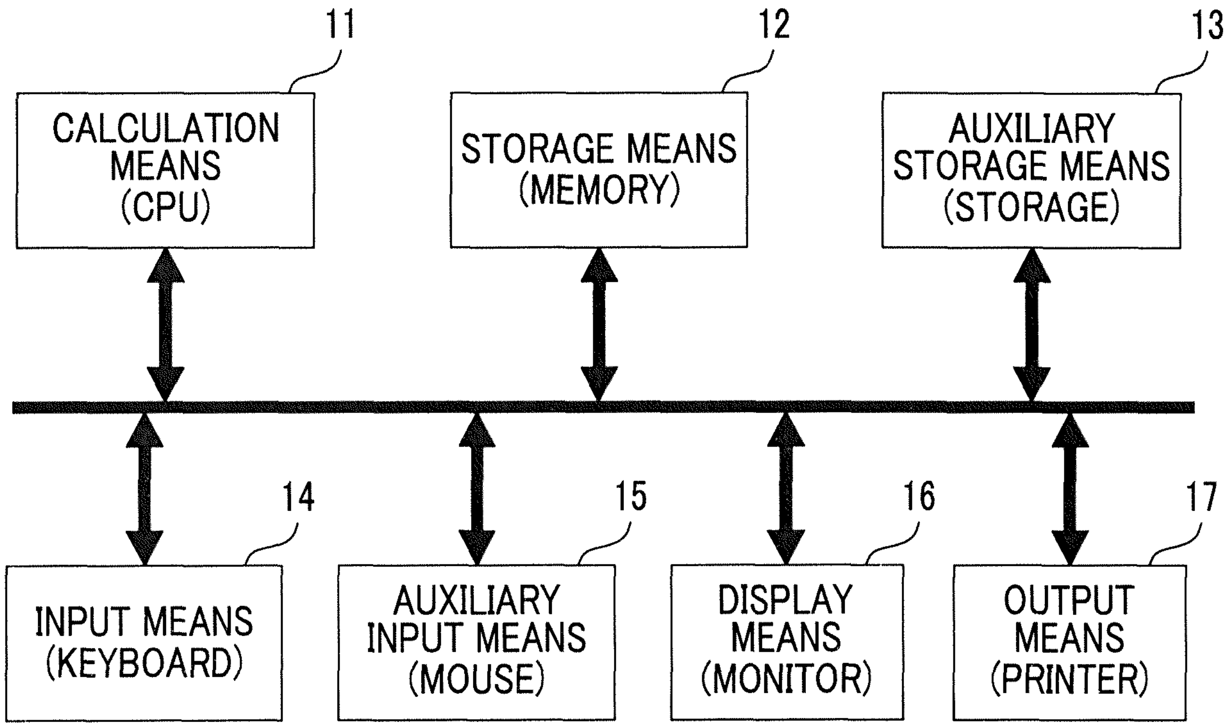
11
12
13
CALCULATION MEANS (CPU)
STORAGE MEANS (MEMORY)
AUXILIARY STORAGE MEANS (STORAGE)
14
15
16
17
INPUT MEANS (KEYBOARD)
AUXILIARY INPUT MEANS (MOUSE)
DISPLAY MEANS (MONITOR)
OUTPUT MEANS (PRINTER)

FIG. 2

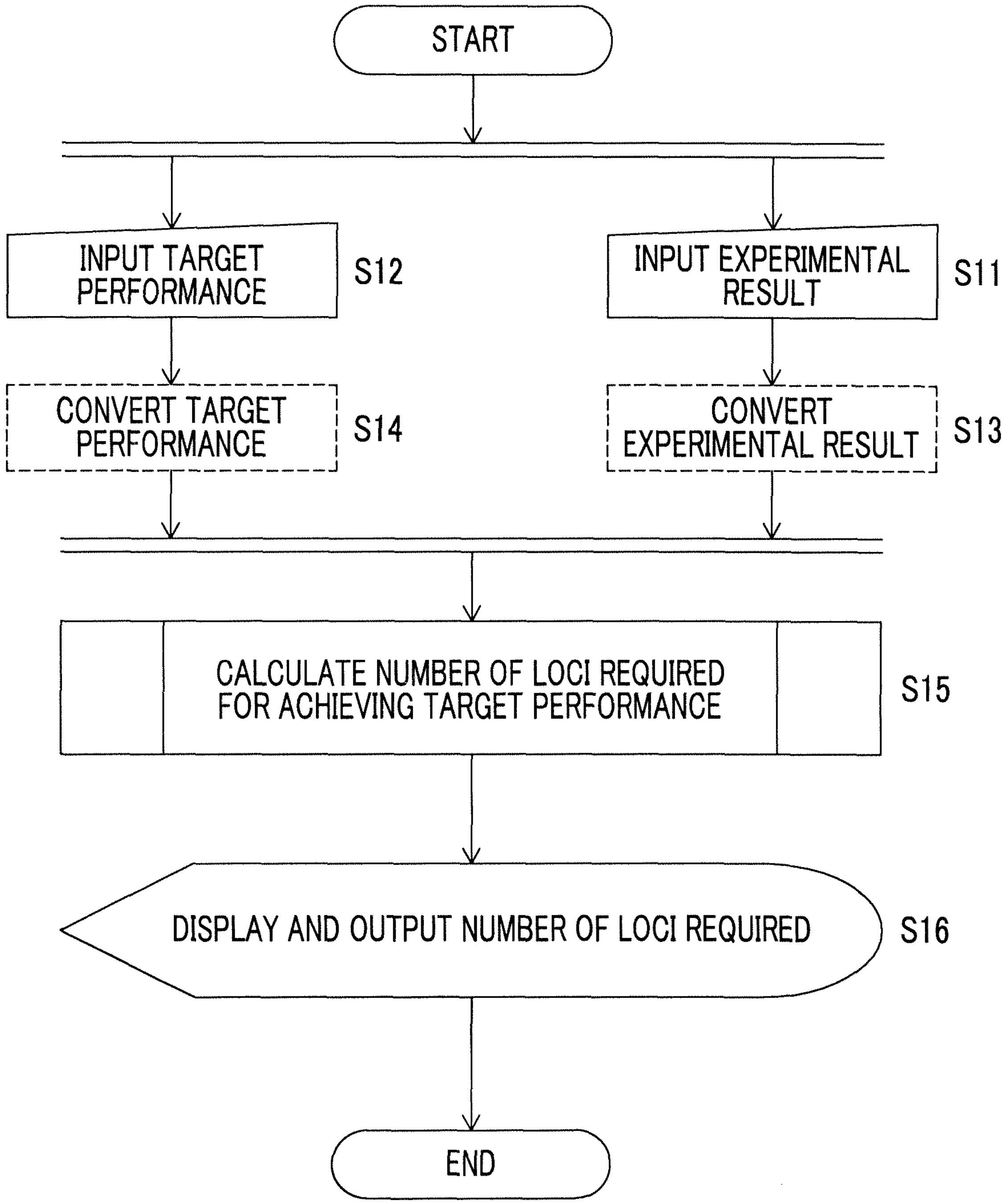
START
INPUT TARGET PERFORMANCE
S12
INPUT EXPERIMENTAL RESULT
S11
CONVERT TARGET PERFORMANCE
S14
CONVERT EXPERIMENTAL RESULT
S13
CALCULATE NUMBER OF LOCI REQUIRED FOR ACHIEVING TARGET PERFORMANCE
S15
DISPLAY AND OUTPUT NUMBER OF LOCI REQUIRED
S16
END

FIG. 3

<table>
<tr><td></td><td></td><td colspan="2">TRUE STATE<br>(CHROMOSOME ANEUPLOIDY)</td><td></td></tr>
<tr><td></td><td></td><td>POSITIVE</td><td>NEGATIVE</td><td></td></tr>
<tr><td rowspan="2">TEST<br>RESULT</td><td>POSITIVE</td><td>TRUE POSITIVE<br>a</td><td>FALSE NEGATIVE<br>(type 1 error)<br>b</td><td>POSITIVE<br>PREDICTIVE VALUE<br>(PPV)<br>a/(a + b)</td></tr>
<tr><td>NEGATIVE</td><td>FALSE NEGATIVE<br>(type 2 error)<br>c</td><td>TRUE NEGATIVE<br>d</td><td>NEGATIVE<br>PREDICTIVE VALUE<br>(NPV)<br>d/(c + d)</td></tr>
<tr><td></td><td></td><td>SENSITIVITY<br>a/(a + c)</td><td>SPECIFICITY<br>d/(b + d)</td><td></td></tr>
<tr><td></td><td></td><td>FALSE<br>NEGATIVE RATE<br>c/(a + c)</td><td>FALSE<br>POSITIVE RATE<br>b/(b + d)</td><td></td></tr>
<tr><td></td><td></td><td colspan="2">PREVALENCE RATE<br>(a + c)/(a + b + c + d)</td><td></td></tr>
</table>

FIG. 4

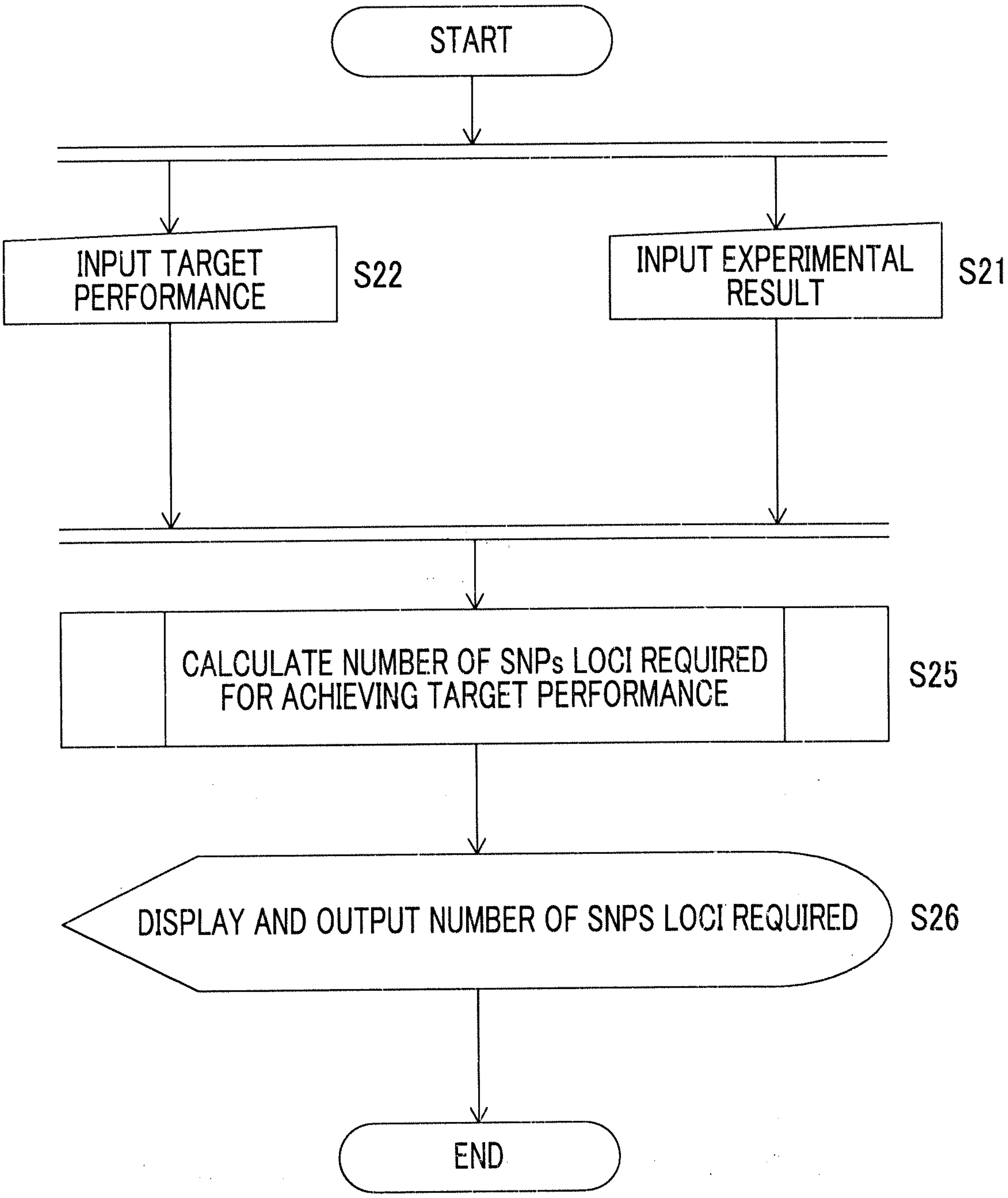
START
INPUT TARGET PERFORMANCE
S22
INPUT EXPERIMENTAL RESULT
S21
CALCULATE NUMBER OF SNPs LOCI REQUIRED FOR ACHIEVING TARGET PERFORMANCE
S25
DISPLAY AND OUTPUT NUMBER OF SNPS LOCI REQUIRED
S26
END

FIG. 5

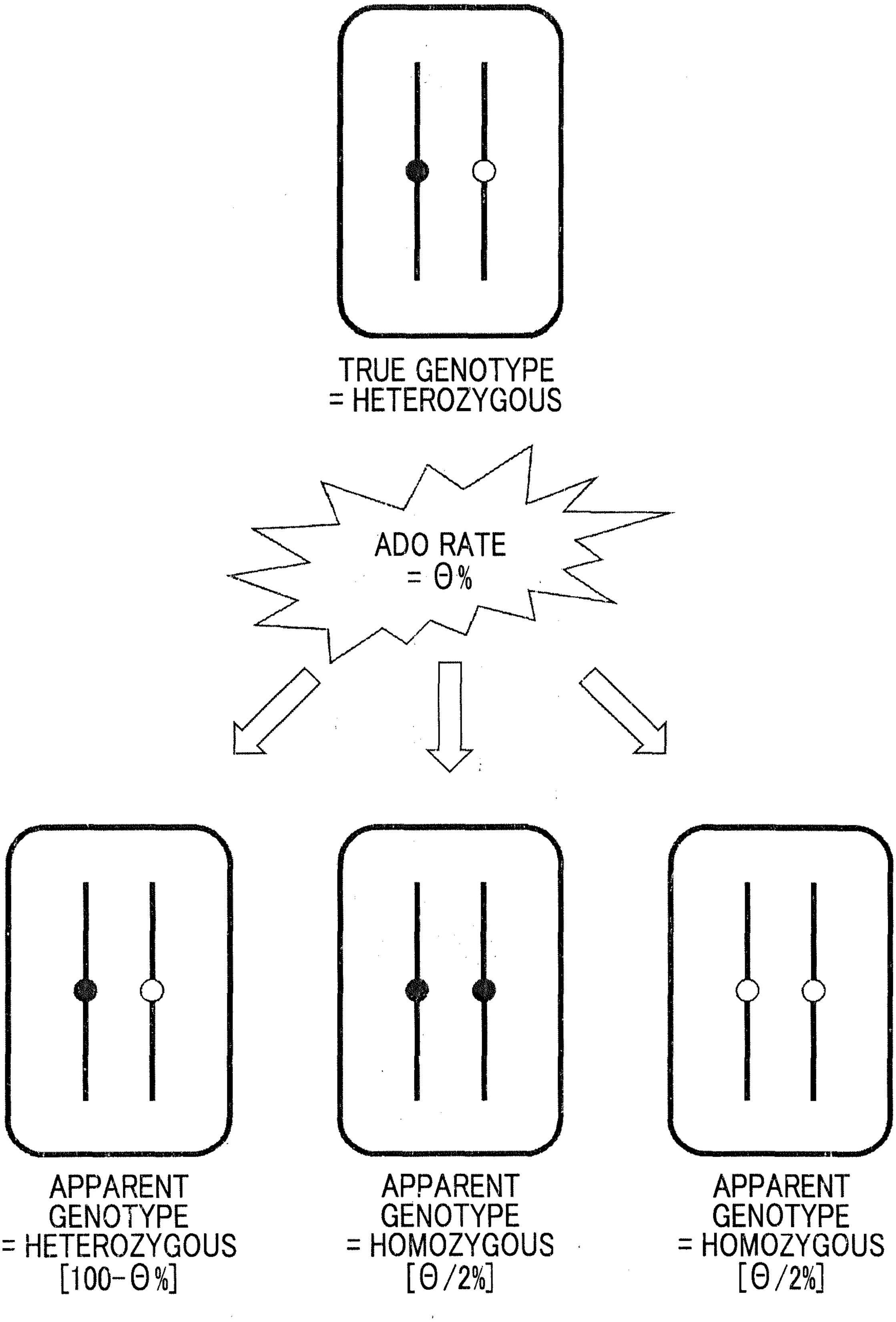
TRUE GENOTYPE
= HETEROZYGOUS
ADO RATE
= Θ%
APPARENT
GENOTYPE
= HETEROZYGOUS
[100-Θ%]
APPARENT
GENOTYPE
= HOMOZYGOUS
[Θ/2%]
APPARENT
GENOTYPE
= HOMOZYGOUS
[Θ/2%]

FIG. 6

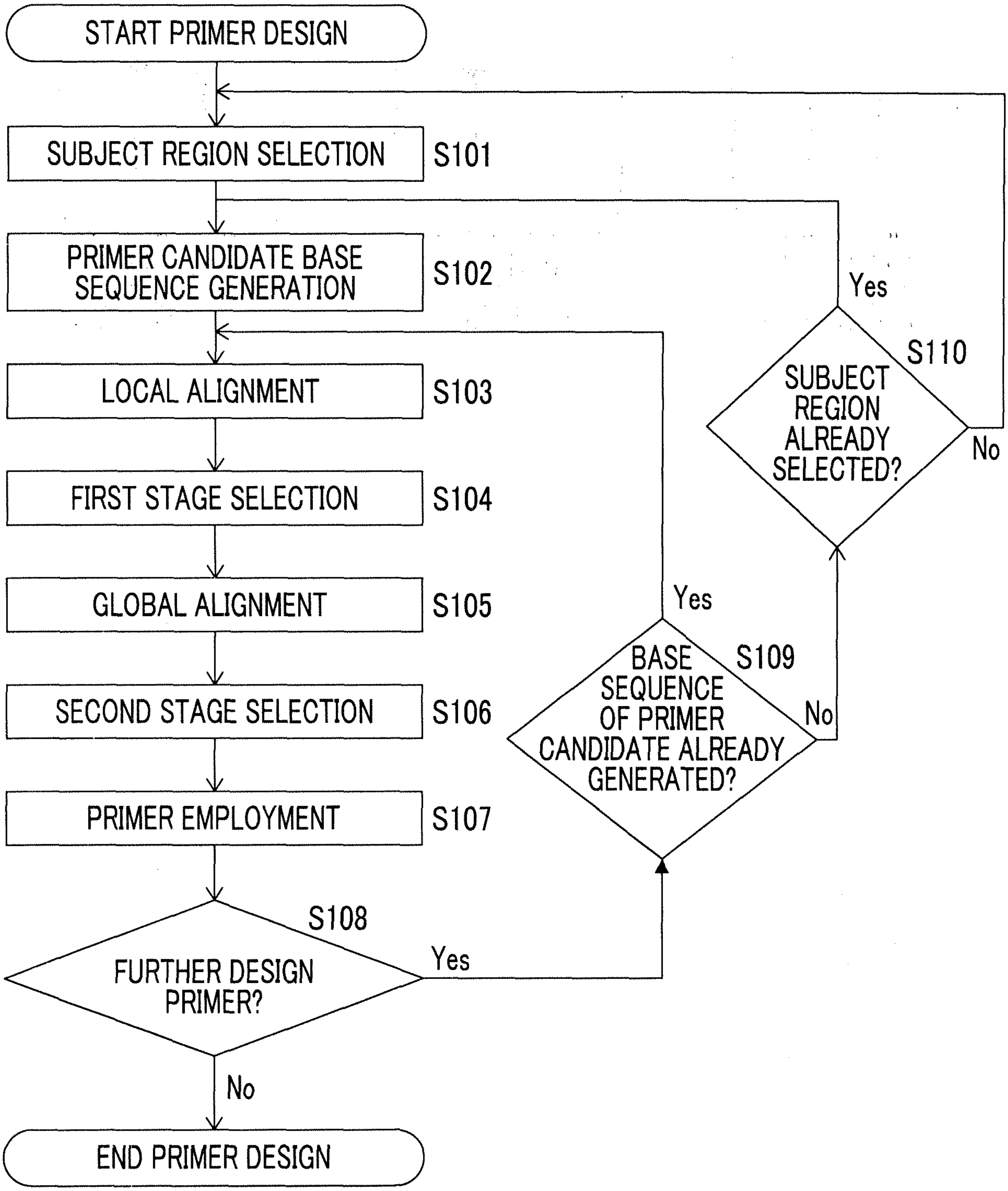
START PRIMER DESIGN
SUBJECT REGION SELECTION
S101
PRIMER CANDIDATE BASE SEQUENCE GENERATION
S102
LOCAL ALIGNMENT
S103
FIRST STAGE SELECTION
S104
GLOBAL ALIGNMENT
S105
SECOND STAGE SELECTION
S106
PRIMER EMPLOYMENT
S107
FURTHER DESIGN PRIMER?
S108
Yes
No
END PRIMER DESIGN
BASE SEQUENCE OF PRIMER CANDIDATE ALREADY GENERATED?
S109
Yes
No
SUBJECT REGION ALREADY SELECTED?
S110
Yes
No

FIG. 7

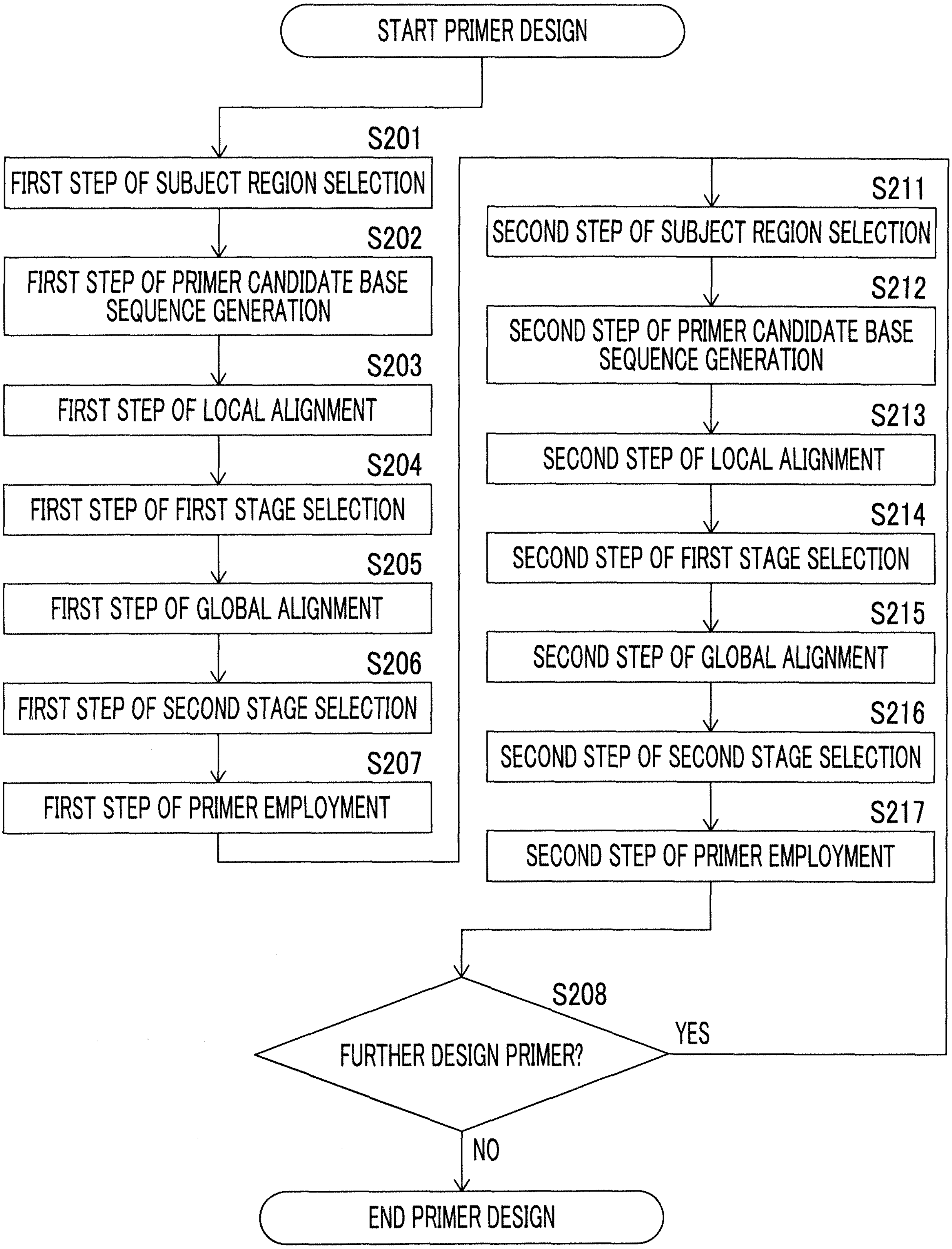
START PRIMER DESIGN
S201
FIRST STEP OF SUBJECT REGION SELECTION
S202
FIRST STEP OF PRIMER CANDIDATE BASE SEQUENCE GENERATION
S203
FIRST STEP OF LOCAL ALIGNMENT
S204
FIRST STEP OF FIRST STAGE SELECTION
S205
FIRST STEP OF GLOBAL ALIGNMENT
S206
FIRST STEP OF SECOND STAGE SELECTION
S207
FIRST STEP OF PRIMER EMPLOYMENT
S211
SECOND STEP OF SUBJECT REGION SELECTION
S212
SECOND STEP OF PRIMER CANDIDATE BASE SEQUENCE GENERATION
S213
SECOND STEP OF LOCAL ALIGNMENT
S214
SECOND STEP OF FIRST STAGE SELECTION
S215
SECOND STEP OF GLOBAL ALIGNMENT
S216
SECOND STEP OF SECOND STAGE SELECTION
S217
SECOND STEP OF PRIMER EMPLOYMENT
S208
FURTHER DESIGN PRIMER?
YES
NO
END PRIMER DESIGN

FIG. 8

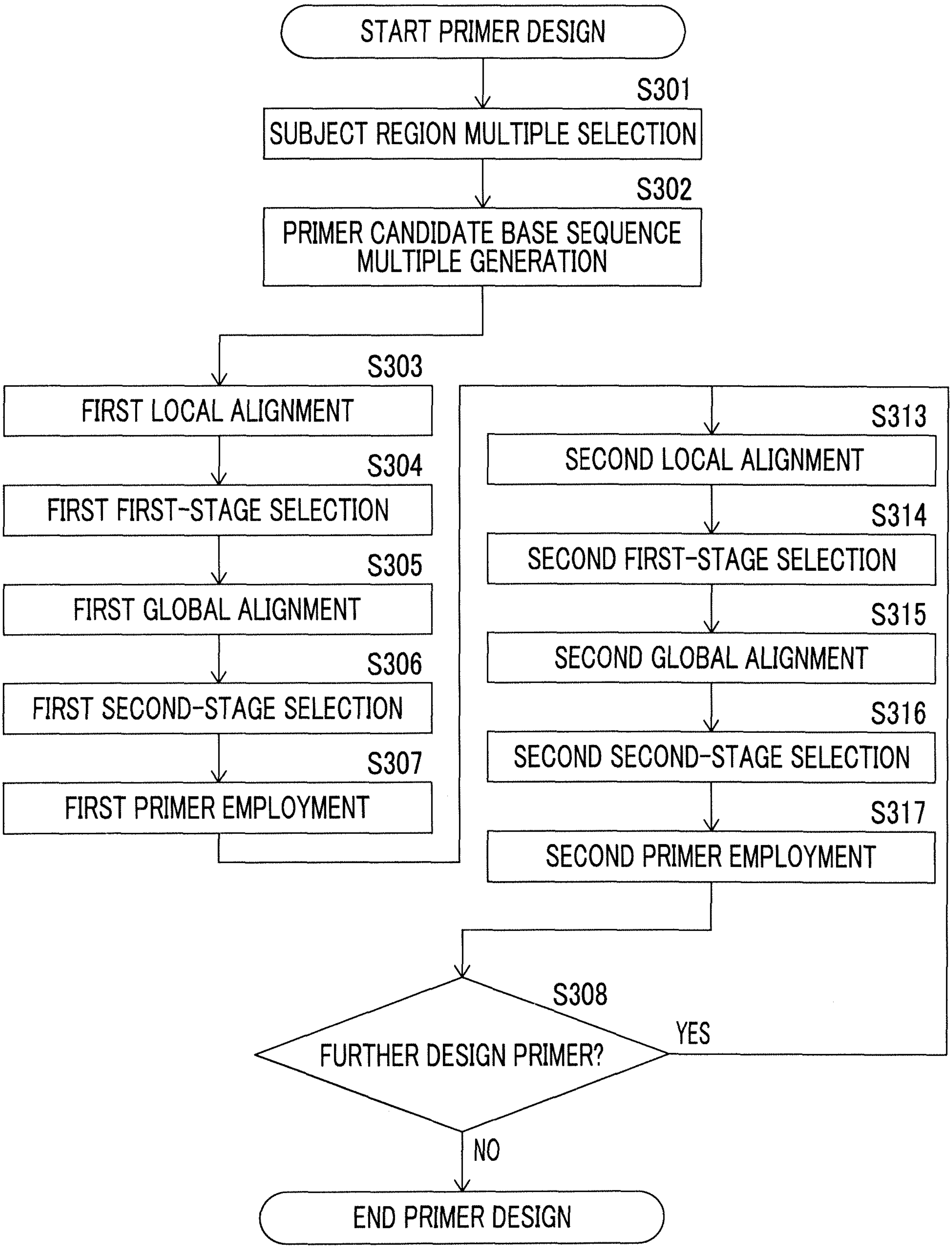
START PRIMER DESIGN
S301
SUBJECT REGION MULTIPLE SELECTION
S302
PRIMER CANDIDATE BASE SEQUENCE MULTIPLE GENERATION
S303
FIRST LOCAL ALIGNMENT
S304
FIRST FIRST-STAGE SELECTION
S305
FIRST GLOBAL ALIGNMENT
S306
FIRST SECOND-STAGE SELECTION
S307
FIRST PRIMER EMPLOYMENT
S313
SECOND LOCAL ALIGNMENT
S314
SECOND FIRST-STAGE SELECTION
S315
SECOND GLOBAL ALIGNMENT
S316
SECOND SECOND-STAGE SELECTION
S317
SECOND PRIMER EMPLOYMENT
S308
FURTHER DESIGN PRIMER?
YES
NO
END PRIMER DESIGN

FIG. 9

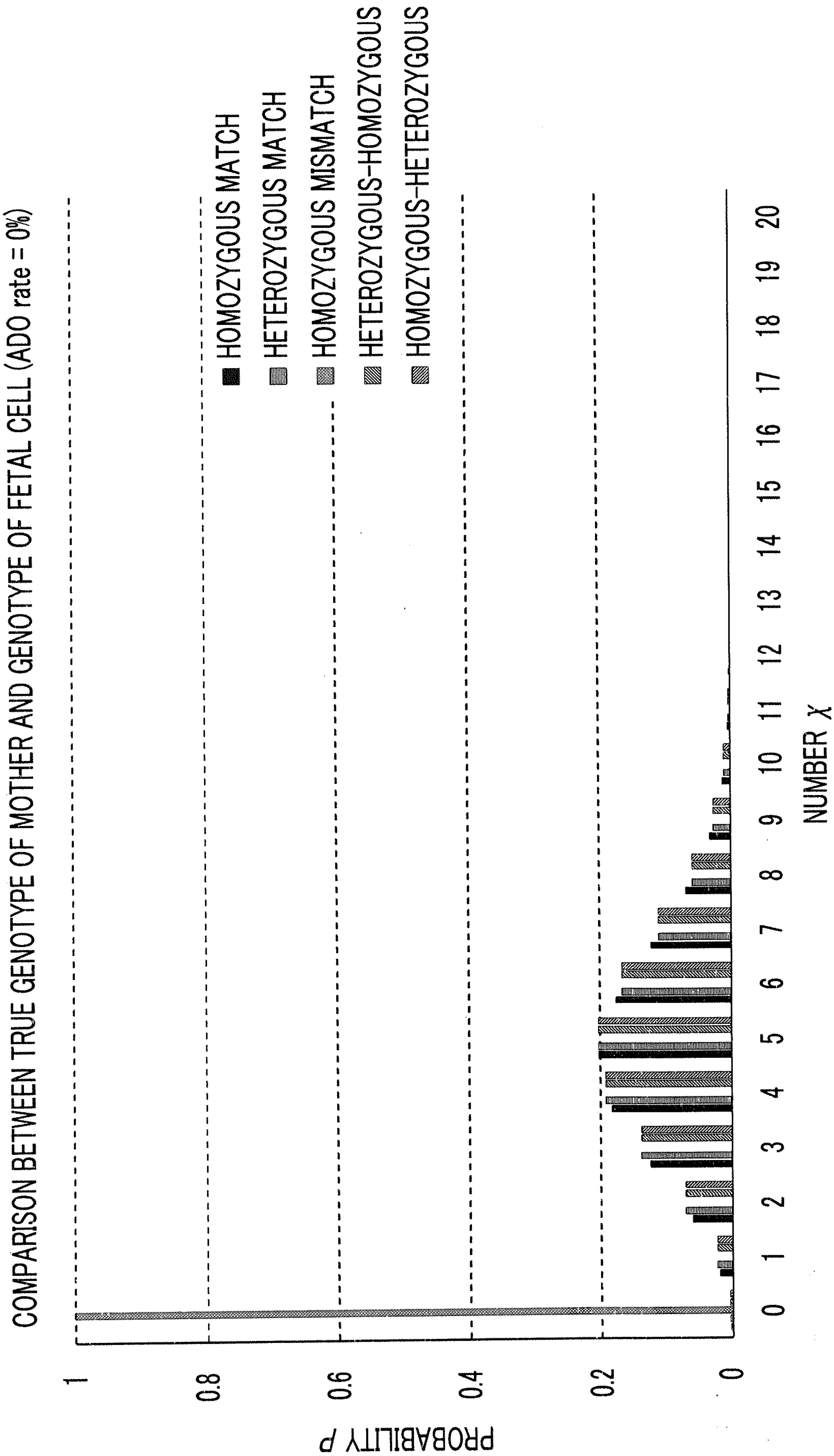
COMPARISON BETWEEN TRUE GENOTYPE OF MOTHER AND GENOTYPE OF FETAL CELL (ADO rate = 0%)
HOMOZYGOUS MATCH
HETEROZYGOUS MATCH
HOMOZYGOUS MISMATCH
HETEROZYGOUS-HOMOZYGOUS
HOMOZYGOUS-HETEROZYGOUS
PROBABILITY P
0
0.2
0.4
0.6
0.8
1
NUMBER χ
0 1 2 3 4 5 6 7 8 9 10 11 12 13 14 15 16 17 18 19 20

FIG. 10

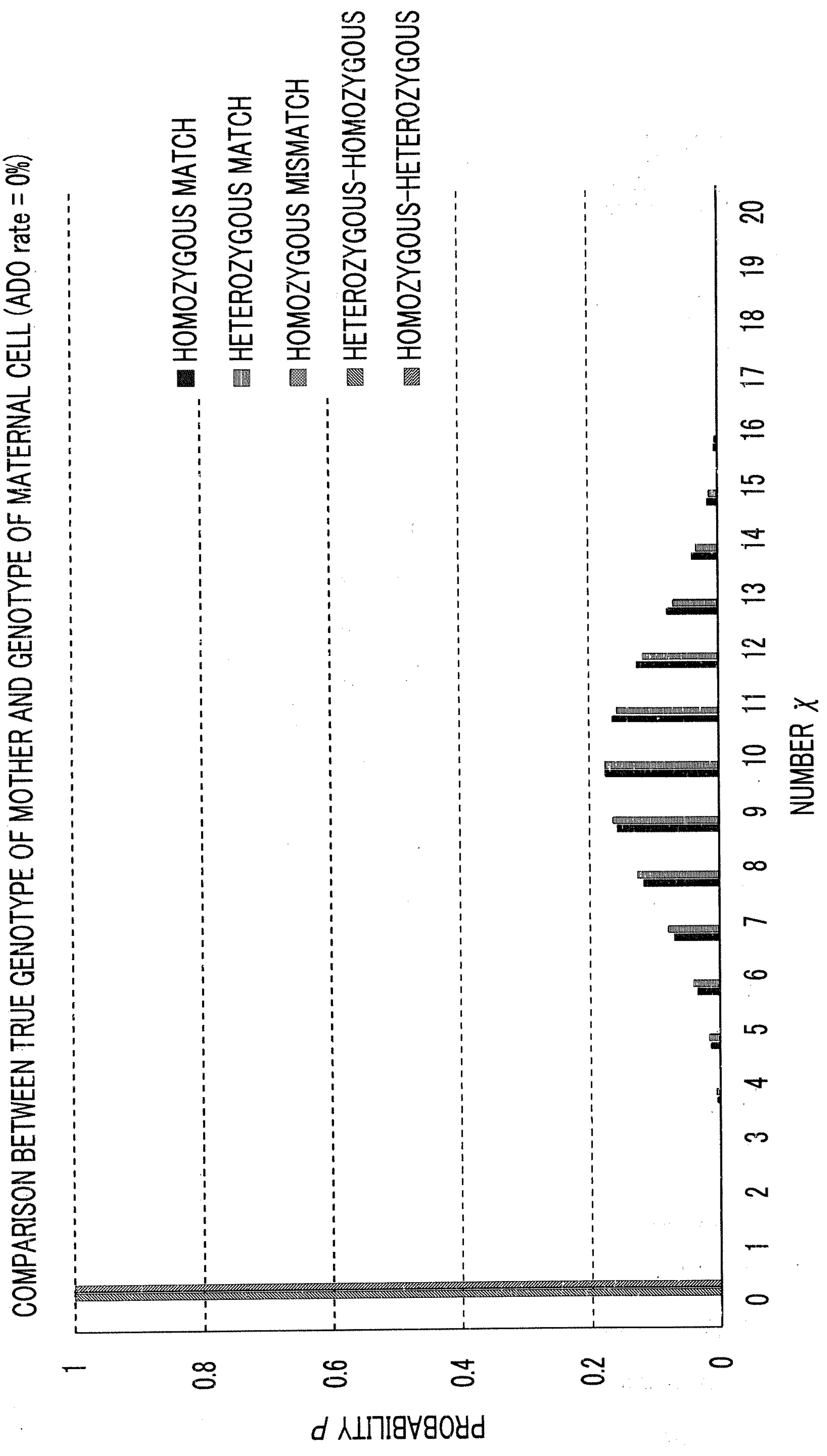
COMPARISON BETWEEN TRUE GENOTYPE OF MOTHER AND GENOTYPE OF MATERNAL CELL (ADO rate = 0%)
HOMOZYGOUS MATCH
HETEROZYGOUS MATCH
HOMOZYGOUS MISMATCH
HETEROZYGOUS-HOMOZYGOUS
HOMOZYGOUS-HETEROZYGOUS
PROBABILITY P
1
0.8
0.6
0.4
0.2
0
NUMBER χ
0
1
2
3
4
5
6
7
8
9
10
11
12
13
14
15
16
17
18
19
20

FIG. 11

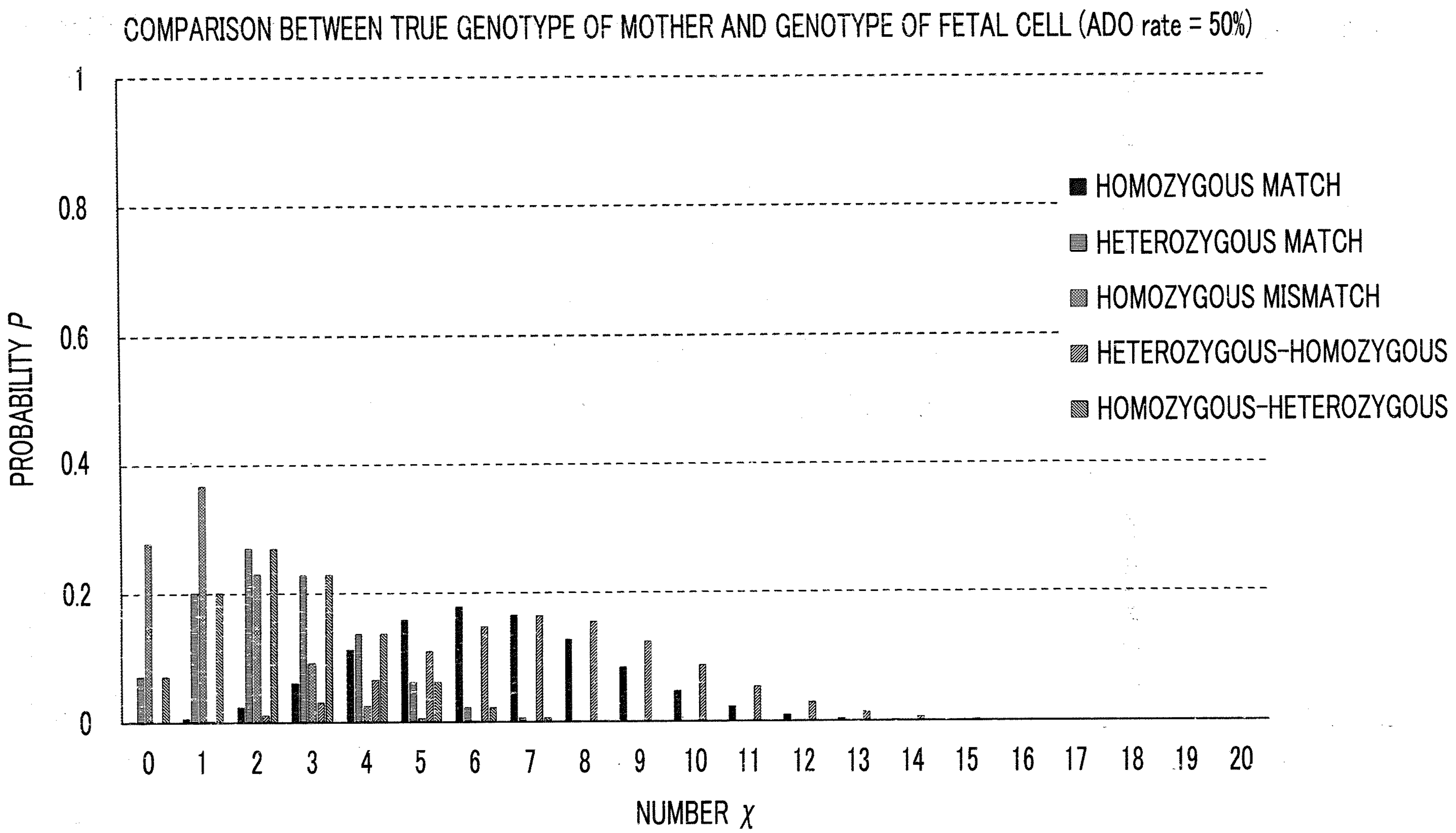
COMPARISON BETWEEN TRUE GENOTYPE OF MOTHER AND GENOTYPE OF FETAL CELL (ADO rate = 50%)
PROBABILITY P
1
0.8
0.6
0.4
0.2
0
HOMOZYGOUS MATCH
HETEROZYGOUS MATCH
HOMOZYGOUS MISMATCH
HETEROZYGOUS-HOMOZYGOUS
HOMOZYGOUS-HETEROZYGOUS
0 1 2 3 4 5 6 7 8 9 10 11 12 13 14 15 16 17 18 19 20
NUMBER χ

FIG. 12

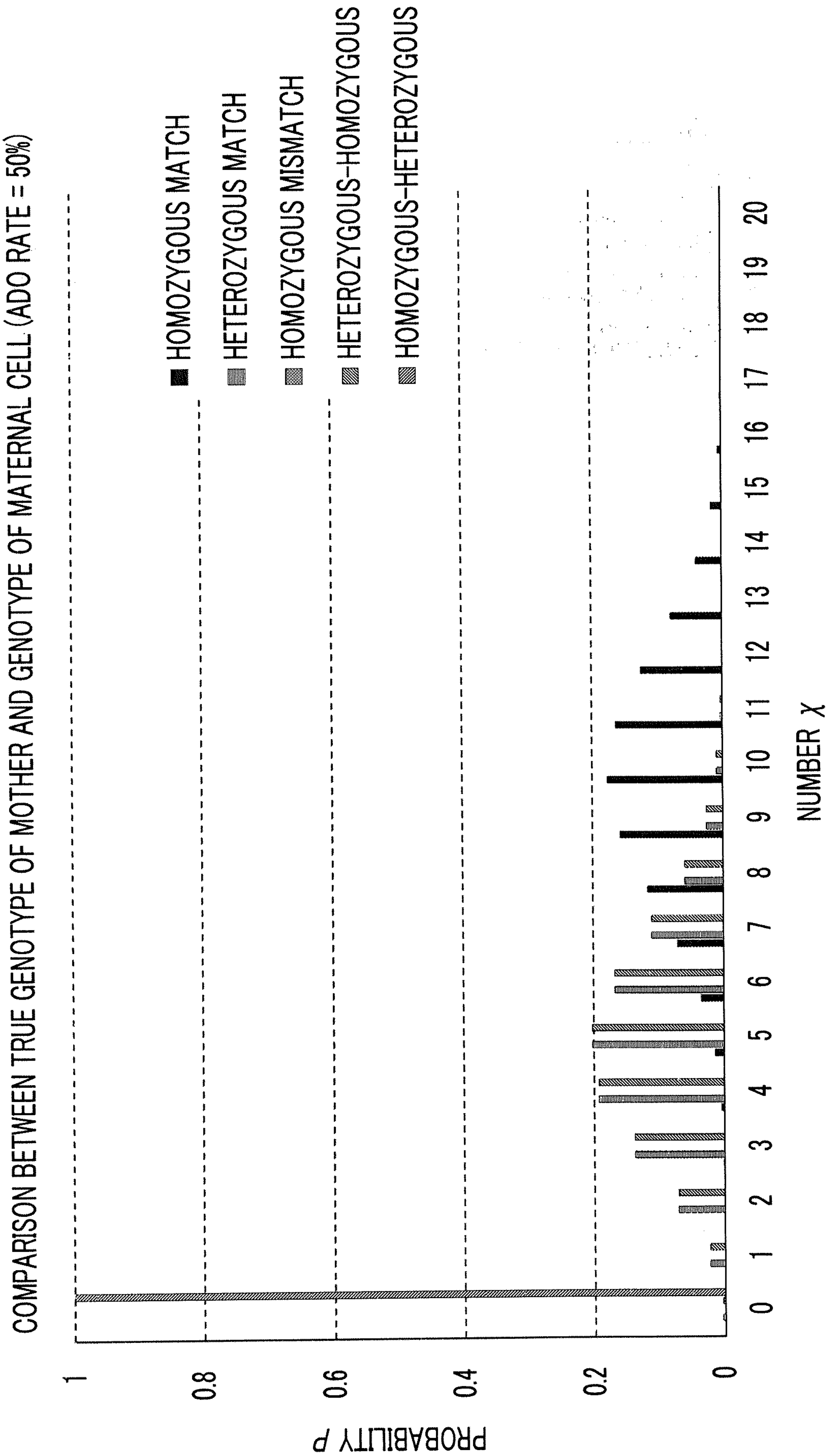
COMPARISON BETWEEN TRUE GENOTYPE OF MOTHER AND GENOTYPE OF MATERNAL CELL (ADO RATE = 50%)
HOMOZYGOUS MATCH
HETEROZYGOUS MATCH
HOMOZYGOUS MISMATCH
HETEROZYGOUS-HOMOZYGOUS
HOMOZYGOUS-HETEROZYGOUS
PROBABILITY P
0
0.2
0.4
0.6
0.8
1
NUMBER χ
0
1
2
3
4
5
6
7
8
9
10
11
12
13
14
15
16
17
18
19
20

FIG. 13

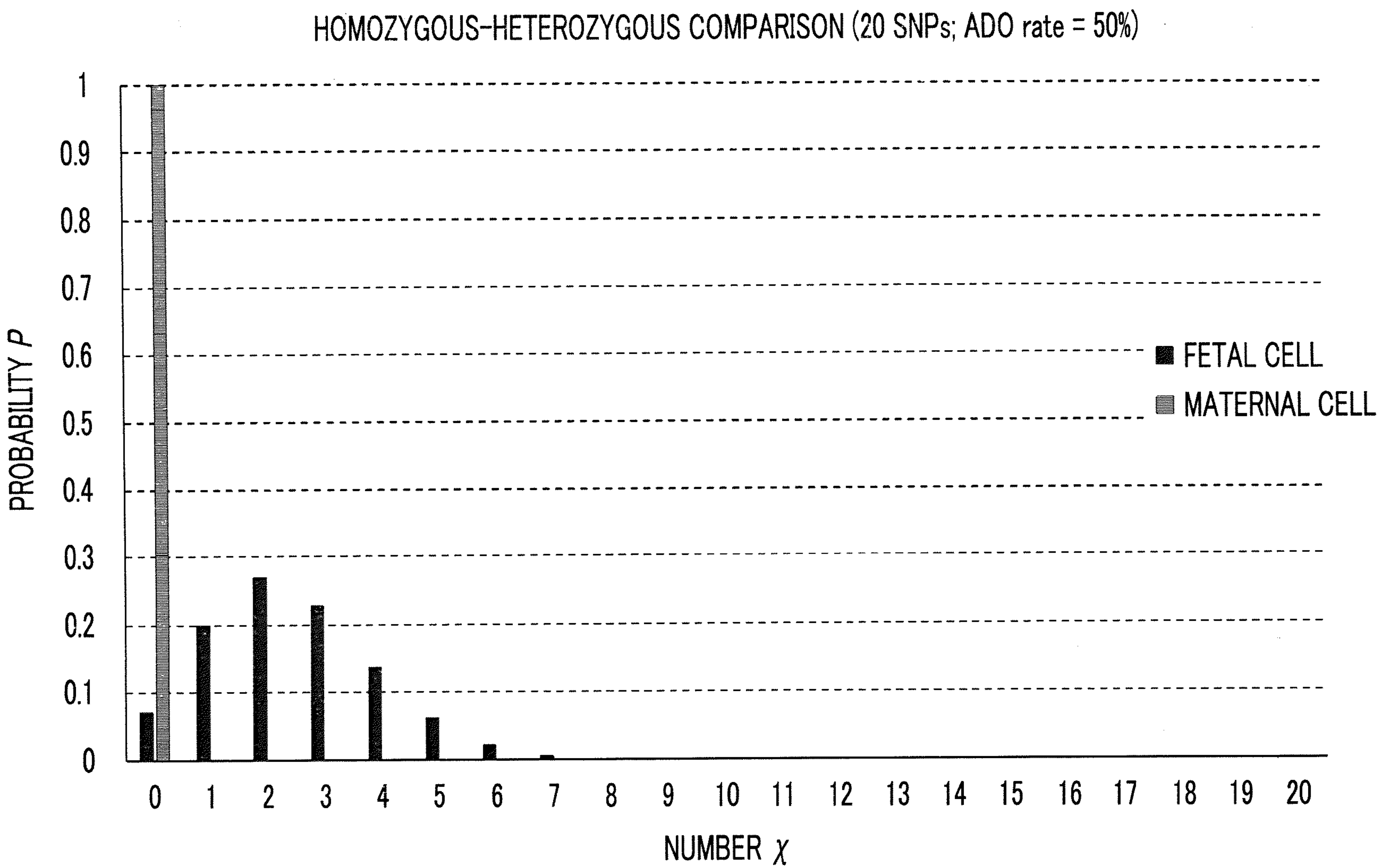
HOMOZYGOUS-HETEROZYGOUS COMPARISON (20 SNPs; ADO rate = 50%)
PROBABILITY P
1
0.9
0.8
0.7
0.6
0.5
0.4
0.3
0.2
0.1
0
FETAL CELL
MATERNAL CELL
0 1 2 3 4 5 6 7 8 9 10 11 12 13 14 15 16 17 18 19 20
NUMBER χ

FIG. 14

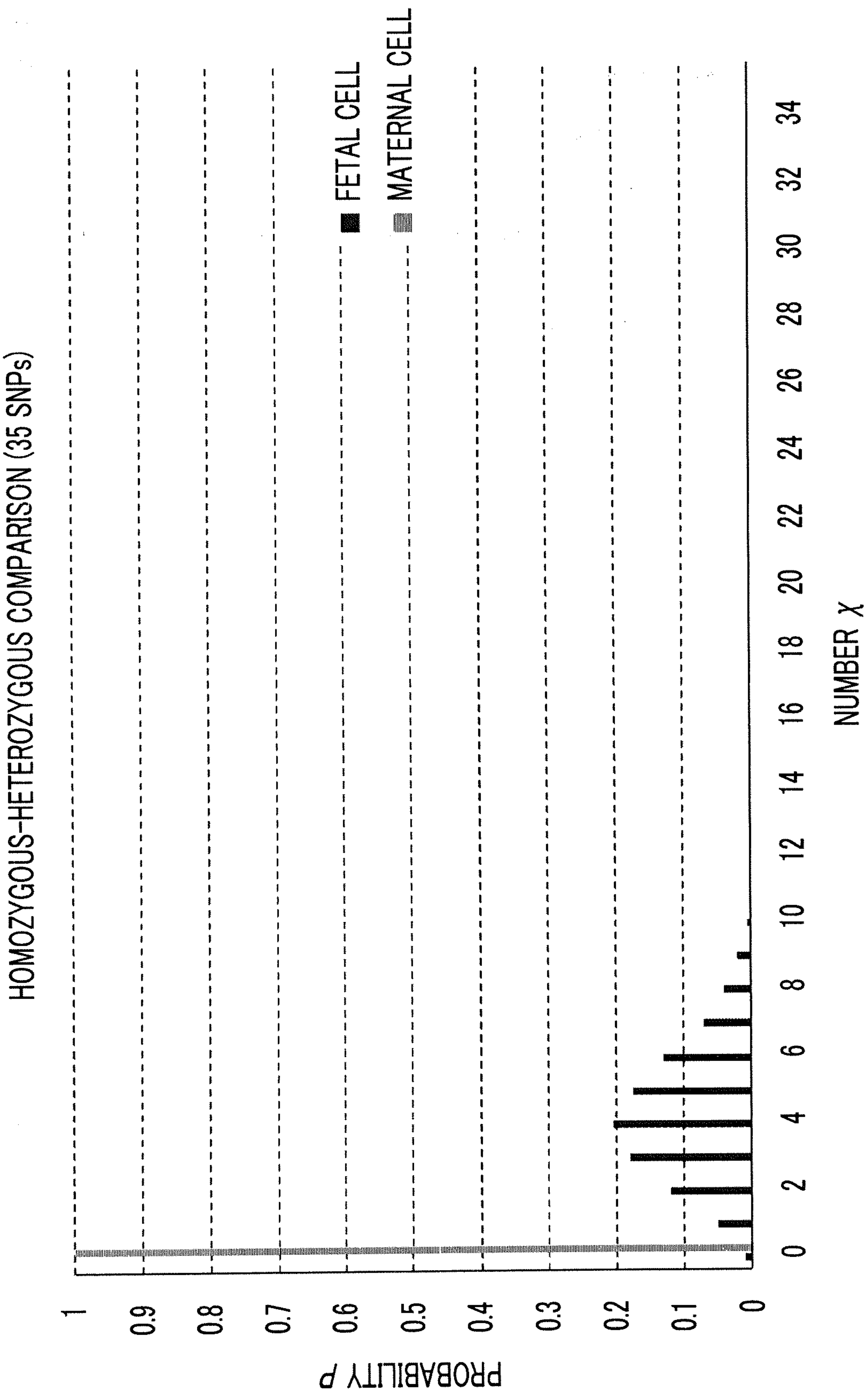
HOMOZYGOUS-HETEROZYGOUS COMPARISON (35 SNPs)
FETAL CELL
MATERNAL CELL
PROBABILITY P
1
0.9
0.8
0.7
0.6
0.5
0.4
0.3
0.2
0.1
0
0
2
4
6
8
10
12
14
16
18
20
22
24
26
28
30
32
34
NUMBER χ

**INTERNATIONAL SEARCH REPORT**

International application No. PCT/JP2017/032699

A. CLASSIFICATION OF SUBJECT MATTER
Int.Cl. C12Q1/68(2006.01)i, G06F19/22(2011.01)i, C12N15/09(2006.01)n

According to International Patent Classification (IPC) or to both national classification and IPC

B. FIELDS SEARCHED

Minimum documentation searched (classification system followed by classification symbols)
Int.Cl. C12Q1/68, G06F19/22, C12N15/09

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model specifications of Japan | 1994-2017 |

Electronic data base consulted during the international search (name of data base and, where practicable, search terms used)
JSTPlus/JMEDPlus/JST7580 (JDreamIII), CAplus/MEDLINE/EMBASE/BIOSIS (STN), WPIDS/WPIX (STN)

C. DOCUMENTS CONSIDERED TO BE RELEVANT

| Category* | Citation of document, with indication, where appropriate, of the relevant passages | Relevant to claim No. |
|---|---|---|
| A | JP 2008-543311 A (STRATAGENE) 04 December 2008, entire text & US 2006/0286558 A1, whole document & WO 2006/138443 A2 & EP 1902064 A2 | 1-9 |
| A | 小林 美和他，ミスマッチ PCR 法を用いた SNPs 解析による母体血中の胎児 DNA 検出の検討，臨床病理，2012, vol. 60, pp. 19-26, (KOBAYASHI, Miwa, et al., SNP-Specific Mismatched PCR for Embryonic DNA Detection Using Blood from Pregnant Mothers, The official journal of Japanese Society of Laboratory Medicine) | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 November 2017 (16.11.2017) | 28 November 2017 (28.11.2017) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2017/032699

C (Continuation). DOCUMENTS CONSIDERED TO BE RELEVANT

| Category* | Citation of document, with indication, where appropriate, of the relevant passages | Relevant to claim No. |
|---|---|---|
| A | JP 2013-530727 A (ESOTERIX GENETIC LAB LLC) 01 August 2013, entire text & US 2012/0021919 A1, whole document & WO 2012/012703 A2 & EP 2596127 A2 | 1-9 |
| A | JP 2013-501514 A (THE CHINESE UNIVERSITY OF HONG KONG) 17 January 2013, entire text & US 2011/0039724 A1, whole document & WO 2011/018600 A1 & EP 2464743 A1 | 1-9 |
| A | JP 2016-067268 A (FUJIFILM CORP.) 09 May 2016, entire text (Family: none) | 1-9 |
| P, A | WO 2017/056931 A1 (FUJIFILM CORP.) 06 April 2017, entire text & JP 2017-63728 A | 1-9 |
| P, A | WO 2017/145739 A1 (FUJIFILM CORP.) 31 August 2017, entire text (Family: none) | 1-9 |
| P, A | WO 2017/145738 A1 (FUJIFILM CORP.) 31 August 2017, entire text (Family: none) | 1-9 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO W6215 A
- JP 17032699 B


### Non-patent literature cited in the description


- **ALTSCHUL, S. A. et al.** Basic Local Alignment Search Tool. *Journal of Molecular Biology,* October 1990, vol. 215, 403-410
- Improved tools for biological sequence comparison. **PEARSON, W. R. et al.** Proceedings of the National Academy of Sciences of the United States of America. National Academy of Sciences, April 1988, vol. 85, 2444-2448